# FASCICULE DE BREVET EUROPEEN

(11) **EP 0 815 214 B1**
(45) Date de publication et mention de la délivrance du brevet: **11.07.2007**
(21) Numéro de dépôt: 96904886.7
(22) Date de dépôt: 21.02.1996
(51) Int. Cl.: C12N 15/10, C12N 15/70, C12N 15/85, C12N 5/10, C12N 1/21, C12Q 1/68, A61K 31/70, A61K 48/00, C12R 1/19

(54) **MOLECULES D'ADN, PREPARATION ET UTILISATION EN THERAPIE GENIQUE**
DNA-MOLEKÜL, HERSTELLUNG UND VERWENDUNG IN DER GENTHERAPIE
DNA MOLECULES, PREPARATION THEREOF AND USE THEREOF IN GENE THERAPY

(30) Priorité: 23.02.1995 FR 9502117
(43) Date de publication de la demande: 07.01.1998
(73) Titulaire: CNRS, 75794 Paris Cedex 16 (FR)
(72) Inventeur: CAMERON, Béatrice, F-75005 Paris (FR); CROUZET, Joel, F-92330 Sceaux (FR); DARQUET, Anne-Marie, F-94400 Vitry-sur-Seine (FR); SCHERMAN, Daniel, F-75645 Paris Cédex 13 (FR); WILS, Pierre, F-75003 Paris (FR)
(74) Mandataire: Texier, Christian
(86) Numéro de dépôt international: PCT/FR1996/000274
(87) Numéro de publication internationale: WO 1996/026270

(56) Documents cités:
- EP-A- 0 300 422
- EP-A- 0 350 341
- WO-A-94/09127
- WO-A-96/05297
- US-A- 5 227 288
- US-A- 5 401 632
- NUCLEIC ACIDS RESEARCH, vol. 20, no. 21, 1992, IRL PRESS LIMITED,OXFORD,ENGLAND, pages 5853-5854, XP002005118 T. TAKABATAKE ET AL.: "The use of purine-rich oligonucleotides in triplex mediated DNA isolation and generation of unidirectional deletions"
- PROC. NATL.ACAD SCI., vol. 89, no. 2, 15 Janvier 1992, NATL. ACAD SCI.,WASHINGTON,DC,US;, pages 495-498, XP002005119 T. ITO ET AL.: "Sequence-specific DNA purification by triplex affinity capture"
- MOLECULAR MICROBIOL., vol. 12, no. 1, 1994, BLACKWELL, OXFORD, GB, pages 131-141, XP002005120 L. EBERL ET AL.: "Analysis of the multimer resolution system encoded by the parCBA operon of broad-host-range plasmid RP4" cité dans la demande
- BIO/TECHNOLOGY, vol. 2, no. 12, Décembre 1984, NATURE PUBL. CO.,NEW YORK, US, pages 1045-1049, XP002005121 K. BACKMAN ET AL.: "Use of synchronous site-specific recombination in vivo to regulate gene expression"
- GENE, vol. 56, 1987, ELSEVIER SCIENCE PUBLISHERS,B.V.,AMSTERDAM,NL;, page 145-151 XP002005122 N. HASAN AND W. SZYBALSKI: "Control of cloned gene expression by promoter inversion in vivo: construction of improved vectors with a multiple cloning site and the ptac promoter" cité dans la demande
- J. BIOL. CHEM. (1994), 269(18), 13511-21 CODEN: JBCHA3;ISSN: 0021-9258, 1994, XP002005123 SU, TIN TIN ET AL: "Selective binding of Escherichia coli RNA polymerase to topoisomers of minicircles carrying the TAC16 and TAC17 promoters"
- NUCLEIC ACIDS RESEARCH, vol. 13, no. 4, 1985, IRL PRESS LIMITED,OXFORD,ENGLAND, pages 1193-1208, XP002005124 M. MIZUUCHI AND K. MIZUUCHI: "The extent of DNA sequence requirement for a functional bacterial attachment site of phage labda"
- TRENDS IN GENETICS, vol. 8, no. 12, Décembre 1992, ELSEVIER SCIENCE LTD., AMSTERDAM, NL, pages 432-439, XP002005125 W. M. STARK ET AL.: "Catalysis by site-specific recombinases" cité dans la demande

### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION: Molécules d'ADN, préparation et utilisation en thérapie génique.
   (iii) NOMBRE DE SEQUENCES: 25
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 37 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 1:
   AATTGTGAAG CCTGCTTTTT TATACTAACT TGAGCGG
   37
(2) INFORMATION POUR LA SEQ ID NO: 2:
   (i) CARACTERISTIQUES DE LA SEQUENCE:
      (A) LONGUEUR: 37 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 2:
   AATTCCGCTC AAGTTAGTAT AAAAAAGCAG GCTTCAC
   37
(2) INFORMATION POUR LA SEQ ID NO: 3:
   (i) CARACTERISTIQUES DE LA SEQUENCE:
      (A) LONGUEUR: 57 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 3:
   GATCCGAAGA AGAAGAAGAA GAAGAAGAAG AAGAAGAAGA AGAAGAAGAA GAAGAAC
   57
(2) INFORMATION POUR LA SEQ ID NO: 4:
   (i) CARACTERISTIQUES DE LA SEQUENCE:
      (A) LONGUEUR: 57 paires de bases
      (B) TYPE: lacide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 4:
   GATCGTTCTT CTTCTTCTTC TTCTTCTTCT TCTTCTTCTT CTTCTTCTTC TTCTTCG
   57
(2) INFORMATION POUR LA SEQ ID NO: 5:
   (i) CARACTERISTIQUES DE LA SEQUENCE:
      (A) LONGUEUR: 25 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 5:
      GAGGCTTCTT CTTCTTCTTC TTCTT 25
(2) INFORMATION POUR LA SEQ ID NO: 6:
   (i) CARACTERISTIQUES DE LA SEQUENCE:
      (A) LONGUEUR: 21 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 6:
   CTGCTTTTTT ATACTAACTT G
   21
(2) INFORMATION POUR LA SEQ ID NO: 7:
   (i) CARACTERISTIQUES DE LA SEQUENCE:
      (A) LONGUEUR: 21 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 7:
   CAGCTTTTTT ATACTAAGTT G 21
(2) INFORMATION POUR LA SEQ ID NO: 8:
   (i) CARACTERISTIQUES DE LA SEQUENCE:
      (A) LONGUEUR: 21 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 8:
   CAGCGCATTC GTAATGCGAA G 21
(2) INFORMATION POUR LA SEQ ID NO: 9:
   (i) CARACTERISTIQUES DE LA SEQUENCE:
      (A) LONGUEUR: 21 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 9:
   CTTATAATTC GTAATGCGAA G 21
(2) INFORMATION POUR LA SEQ ID NO: 10:
   (i) CARACTERISTIQUES DE LA SEQUENCE:
      (A) LONGUEUR: 19 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 10:
   AACACTTTCT TAAATGGTT 19
(2) INFORMATION POUR LA SEQ ID NO: 11:
   (i) CARACTERISTIQUES DE LA SEQUENCE:
      (A) LONGUEUR: 19 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 11:
   AACACTTTCT TAAATTGTC 19
(2) INFORMATION POUR LA SEQ ID NO: 12:
   (i) CARACTERISTIQUES DE LA SEQUENCE:
      (A) LONGUEUR: 19 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 12:
   AAGGGATTTA AAATCCCTC 19
(2) INFORMATION POUR LA SEQ ID NO: 13:
   (i) CARACTERISTIQUES DE LA SEQUENCE:
      (A) LONGUEUR: 19 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 13:
   ATGGTATTTA AAATCCCTC 19
(2) INFORMATION POUR LA SEQ ID NO: 14:
   (i) CARACTERISTIQUES DE LA SEQUENCE:
      (A) LONGUEUR: 49 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 14:
   TTCTCTGTCG GGGTGGCGGG ATTTGAACCC ACGACCTCTT CGTCCCGAA 49
(2) INFORMATION POUR LA SEQ ID NO: 15:
   (i) CARACTERISTIQUES DE LA SEQUENCE:
      (A) LONGUEUR: 27 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 15:
   CGTCGAAATA TTATAAATTA TCAGACA
   27
(2) INFORMATION POUR LA SEQ ID NO: 16:
   (i) CARACTERISTIQUES DE LA SEQUENCE:
      (A) LONGUEUR: 66 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 16:
   GATCTGAAGA AGAAGAAGAA GAAGAAGAAG AAGAAGAAGA AGAAGAAGAA GAAGAACTGC
   60
   AGATCT
   66
(2) INFORMATION POUR LA SEQ ID NO: 17:
   (i) CARACTERISTIQUES DE LA SEQUENCE:
      (A) LONGUEUR: 66 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEO ID NO: 17:
   GATCAGATCT GCAGTTCTTC TTCTTCTTCT TCTTCTTCTT CTTCTTCTTC TTCTTCTTCT
   60
   TCTTCA
   66
(2) INFORMATION POUR LA SEQ ID NO: 18:
   (i) CARACTERISTIQUES DE LA SEQUENCE:
      (A) LONGUEUR: 58 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 18:
(2) INFORMATION POUR LA SEQ ID NO: 19:
   (i) CARACTERISTIQUES DE LA SEQUENCE:
      (A) LONGUEUR: 21 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 19:
   CTTCTTCTTC TTCTTCTTCT T
   21
(2) INFORMATION POUR LA SEQ ID NO: 20:
   (i) CARACTERISTIQUES DE LA SEQUENCE:
      (A) LONGUEUR: 56 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 20:
   AGCTTCTCGA GCTGCAGGAT ATCGAATTCG GATCCTCTAG AGCGGCCGCG AGCTCC 56
(2) INFORMATION POUR LA SEQ ID NO: 21:
   (i) CARACTERISTIQUES DE LA SEQUENCE:
      (A) LONGUEUR: 56 paires de bases
      (B) TYPE: lacide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 21:
   AGCTGGAGCT CGCGGCCGCT CTAGAGGATC CGAATTCGAT ATCCTGCAGC TCGAGA 56
(2) INFORMATION POUR LA SEQ ID NO: 22:
   (i) CARACTERISTIQUES DE LA SEQUENCE:
      (A) LONGUEUR: 49 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 22:
   ACTAGTGGCC ATGCATCCGC TCAAGTTAGT ATAAAAAAGC AGGCTTCAG 49
(2) INFORMATION POUR LA SEQ ID NO: 23:
   (i) CARACTERISTIQUES DE LA SEQUENCE:
      (A) LONGUEUR: 57 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 23:
   AGCTCTGAAG CCTGCTTTTT TATACTAACT TGAGCGGATG CATGGCCACT AGTAGCT
   57
(2) INFORMATION POUR LA SEQ ID NO: 24:
   (i) CARACTERISTIQUES DE LA SEQUENCE:
      (A) LONGUEUR: 29 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 24:
   GCGTCTAGAA CAGTATCGTG ATGACAGAG
   29
(2) INFORMATION POUR LA SEQ ID NO: 25:
   (i) CARACTERISTIQUES DE LA SEQUENCE:
      (A) LONGUEUR: 29 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 25:
   GCCAAGCTTA GCTTTGCACT GGATTGCGA
   29

## Description

La thérapie génique consiste à corriger une déficience ou une anornalie en introduisant une information génétique dans la cellule ou l'organe affecté. Cette information peut être introduite soit in vitro dans une cellule extraite de l'organe et ensuite réinjectée dans l'organisme, soit in vivo, directement dans le tissu visé. S'agissant d'une molécule de haut poids moléculaire et de charge négative, l'ADN a des difficultés pour traverser spontanément les membranes cellulaires phospholipidiques. Différents vecteurs sont donc utilisés afin de permettre le transfert de gène : les vecteurs viraux d'une part, les vecteurs chimiques et/ou biochimiques, naturels ou synthétiques, d'autre part. Les vecteurs viraux (rétrovirus, adénovirus, virus adéno-associés,...) sont très efficaces, notamment pour le passage des membranes, mais présentent un certain nombre de risques tels que la pathogénicité, la recombinaison, la réplication, l'immunogénicité, ... Les vecteurs chimiques et/ou biochimiques permettent d'éviter ces risques (pour revues, voir Behr, 1993, Cotten et Wagner, 1993). Ce sont par exemple des cations (phosphate de calcium, DEAE-dextran,...) qui agissent en formant des précipités avec l'ADN, lesquels peuvent être "phagocytés" par les cellules. D peut également s'agir de liposomes dans lesquels l'ADN est incorporé et qui fusionnent avec la membrane plasmique. Les vecteurs synthétiques de transfert de gènes sont généralement des lipides ou des polymères cationiques qui complexent l'ADN et forment avec lui une particule portant des charges positives en surface. Ces particules sont capables d'interagir avec les charges négatives de la membrane cellulaire, puis de franchir celle-ci. On peut citer comme exemples de tels vecteurs le dioctadécylamidoglycylspermine (DOGS, Transfectam^{™}) ou le chlorure de N-[1-(2,3-dioleyloxy)propyl]-N,N,N-triméthylarnmonium (DOTMA, Lipofectin^{™}). Des protéines chimères ont aussi été développées : elles sont constituées d'une partie polycationique qui condense l'ADN, liée à un ligand qui se fixe sur un récepteur membranaire et entraîne le complexe dans les cellules par endocytose. Il est ainsi théoriquement possible de "cibler" un tissu ou certaines populations cellulaires, afin d'améliorer la biodisponibilité in vivo du gène transféré.

Toutefois, l'utilisation de vecteurs chimiques et/ou biochimiques ou d'ADN nu implique la possibilité de produire des quantités importantes d'ADN de pureté pharmacologique. En effet, dans ces techniques de thérapie génique, le médicament est constitué par l'ADN même et il est essentiel de pouvoir fabriquer, dans des quantités adaptées, des ADN ayant des propriétés appropriées à un usage thérapeutique chez l'homme.

Les plasmides utilisés actuellement en thérapie génique portent (i) une origine de réplication, (ii) un gène marqueur tel qu'un gène de résistance à un antibiotique (kanamycine, ampicilline...) et (iii) un ou plusieurs transgènes avec des séquences nécessaires à leur expression (enhanceur(s), promoteur(s), séquences de polyadénylation...). Ces plasmides, utilisés actuellement en thérapie génique (au niveau d'essais cliniques tels que le traitement de mélanomes, Nabel et al., 1992, ou au niveau d'études expérimentales), présentent cependant certains inconvénients, liés notamment à leur dissémination dans l'organisme. Ainsi, en raison de cette dissémination, une bactérie compétente présente dans l'organisme peut, à une fréquence faible, recevoir ce plasmide. Ceci a d'autant plus de chances de se passer qu'il s'agit d'un traitement de thérapie génique in vivo dans lequel l'ADN peut être disséminé dans l'organisme du patient et peut se trouver au contact de bactéries qui infectent ce patient ou bien de bactéries de la flore commensale. Si la bactérie receveuse du plasmide est une entérobactérie, telle qu'E. coli, ce plasmide peut se répliquer. Un tel événement conduit alors à la dissémination du gène thérapeutique. Dans la mesure où les gènes thérapeutiques utilisés dans des traitements de thérapie génique peuvent coder par exemple pour une lymphokine, un facteur de croissance, un antioncogène, ou une protéine dont la fonction fait défaut chez l'hôte et permet donc de corriger un défaut génétique, la dissémination de certains de ces gènes pourrait avoir des effets imprévisibles et préoccupants (par exemple si une bactérie pathogène acquérait le gène d'un facteur de croissance humain). De plus, les plasmides utilisés en thérapie génique non virale possèdent aussi un marqueur de résistance à un antibiotique (ampicilline, kanamycine...). La bactérie acquérant un tel plasmide a donc un avantage sélectif indéniable puisque tout traitement antibiothérapique, utilisant un antibiotique de la même famille que celui sélectionnant le gène de résistance du plasmide, va conduire à la sélection du plasmide en question. A cet égard, l'ampicilline fait partie des β-lactames qui est la famille d'antibiotiques les plus utilisés au monde. Il est donc nécessaire de chercher à limiter au maximum la dissémination des gènes thérapeutiques et des gènes de résistance. Par ailleurs, les gènes portés par le plasmide, correspondants à la partie vecteur du plasmide (fonction(s) nécessaires(s) à la réplication, gène de résistance) risquent également d'être exprimés dans les cellules transfectées. Il existe en effet un bruit de fond de transcription, que l'on ne peut exclure, dû aux signaux d'expression de l'hôte sur le plasmide. Cette expression de protéines exogènes peut être tout à fait préjudiciable dans un certain nombre de traitements de thérapie génique, du fait de leur immunogénicité potentielle et donc de l'attaque par le système immunitaire des cellules transfectées.

Il est donc particulièrement important de pouvoir disposer de molécules d'ADN médicament ayant une pureté génétique appropriée à un usage thérapeutique. II est également particulièrement important de disposer de méthodes permettant de préparer ces molécules d'ADN dans des quantités adaptées à un usage pharmaceutique. La présente invention apporte une solution à ces problèmes.

La présente invention décrit en effet des molécules d'ADN, utilisables en thérapie génique, ayant une pureté génétique très améliorée et de grandes propriétés de biodisponibilité. L'invention décrit également une méthode particulièrement efficace pour la préparation de ces molécules, et pour leur purification.

La présente invention réside notamment dans la mise au point de molécules d'ADN utilisables en thérapie génique, pratiquement dépourvues de toute région non-thérapeutique. Les molécules d'ADN selon l'invention, également désignées minicercles en raison de leur structure circulaire, de leur taille réduite et de leur forme superenroulée, présentent de nombreux avantages.

Elles permettent tout d'abord d'éliminer les risques liés à la dissémination du plasmide, tels que (1) la réplication et la dissémination, pouvant entraîner une surexpression non contrôlée du gène thérapeutique, (2) la dissémination et l'expression de gènes de résistance, (3) l'expression de gènes présents dans la partie non-thérapeutique du plasmide, potentiellement immunogènes et/ou inflammatoires, etc. L'information génétique contenue dans les molécules d'ADN selon l'invention se limite en effet essentiellement au(x) gène(s) thérapeutique(s) et aux signaux de régulation de son (leur) expression (ni origine de réplication, ni gène de résistance à un antibiotique, etc). La probabilité que ces molécules (et donc l'information génétique qu'elles contiennent) soient transférées à un microorganisme, et maintenues de manière stable, est quasiment nulle.

De plus, en raison de leur taille réduite, les molécules d'ADN selon l'invention ont potentiellement une meilleure biodisponibilité in vivo. En particulier, elles présentent des capacités de pénétration et de distribution cellulaires améliorées. Ainsi, il est reconnu que le coefficient de diffusion dans les tissus est inversement proportionnel au poids moléculaire (Jain. 1987). De même, au niveau cellulaire, les molécules de haut poids moléculaire ont une moins bonne perméabilité à travers la membrane plasmique. En outre, pour le passage du plasmide au noyau, indispensable à son expression, le poids moléculaire élevé est également un inconvénient, les pores nucléaires imposant une limite de taille pour la diffusion vers le noyau (Landford et al., 1986). L'élimination des parties non-thérapeutiques du plasmide (origine de réplication et gène de résistance notamment) selon l'invention permet également de diminuer la taille des molécules d'ADN. Cette diminution peut être estimée à un facteur 2. en comptant par exemple 3 kb pour l'origine de réplication et le marqueur de résistance (partie vecteur) et 3 kb pour le transgène avec les séquences nécessaires à son expression. Cette diminution (i) de poids moléculaire et (ii) de charge négative, confère aux molécules de l'invention des capacités améliorées de diffusion et de biodisponibilité tissulaires, cellulaires et nucléaires.

Un premier objet de l'invention réside donc dans une molécule d'ADN double brin ayant les caractéristiques suivantes :
Molécule d'ADN double brin **caractérisée en ce que**:
- elle est sous forme circulaire et super enroulée,
- elle comprend une cassette d'expression constituée d'un gène d'intérêt thérapeutique, vaccinal, agronomique ou vétérinaire, sous contrôler d'un promoteur et d'un terminateur transcriptionnels actifs dans les cellules mammifères,
- elle est dépourvue d'origine de réplication
- elle est dépourvue de gène marqueur, et,
- elle comprend une région résultant de la recombinaison site spécifique entre deux séquences, ladite région étant située hors de la cassette d'expression.
Comme indiqué ci-avant, les molécules de l'invention sont essentiellement dépourvues de régions non-thérapeutiques, et en particulier d'origine de réplication et/ou de gène marqueur.

La présente invention découle également de la mise au point d'un procédé, de constructions et d'hôtes cellulaires spécifiques, particulièrement efficaces pour la production de ces molécules d'ADN thérapeutiques. Plus particulièrement, le procédé selon l'invention réside dans la production des molécules d'ADN thérapeutiques définies ci-avant par excision à partir d'un plasmide ou d'un chromosome par recombinaison site-spécifique. Le procédé selon l'invention est particulièrement avantageux puisqu'il ne nécessite pas d'étape de purification préalable du plasmide, est très spécifique, particulièrement efficace, ne diminue pas les quantités d'ADN produites et conduit directement à des molécules thérapeutiques d'une très grande pureté génétique et d'une grande biodisponibilité. Ce procédé conduit en effet à la génération de molécules d'ADN circulaires (minicercles) contenant essentiellement le gène d'intérêt et les séquences régulatrices permettant son expression dans les cellules, le tissu, l'organe, ou l'appareil, ou même l'organisme entier où l'expression est souhaitée. En outre, ces molécules peuvent ensuite être purifiées par des techniques classiques.

La recombinaison site-spécifique peut être réalisée grâce à divers systèmes qui entraînent la recombinaison site-spécifique entre des séquences. Plus préférentiellement, la recombinaison site-spécifique dans le procédé de l'invention est obtenue au moyen de deux séquences spécifiques qui sont capables de recombiner entre elles en présence d'une protéine spécifique, généralement désignée recombinase. Pour cette raison, les molécules d'ADN selon l'invention comprennent généralement en outre une séquence résultant de cette recombinaison site spécifique. Les séquences permettant la recombinaison utilisées dans le cadre de l'invention comprennent généralement de 5 à 100 paires de bases, et, plus préférentiellement, moins de 50 paires de bases.

La recombinaison site-spécifique peut être réalisée in vivo (c'est à dire dans la cellule hôte) ou in vitro (c'est à dire sur une préparation de plasmide).

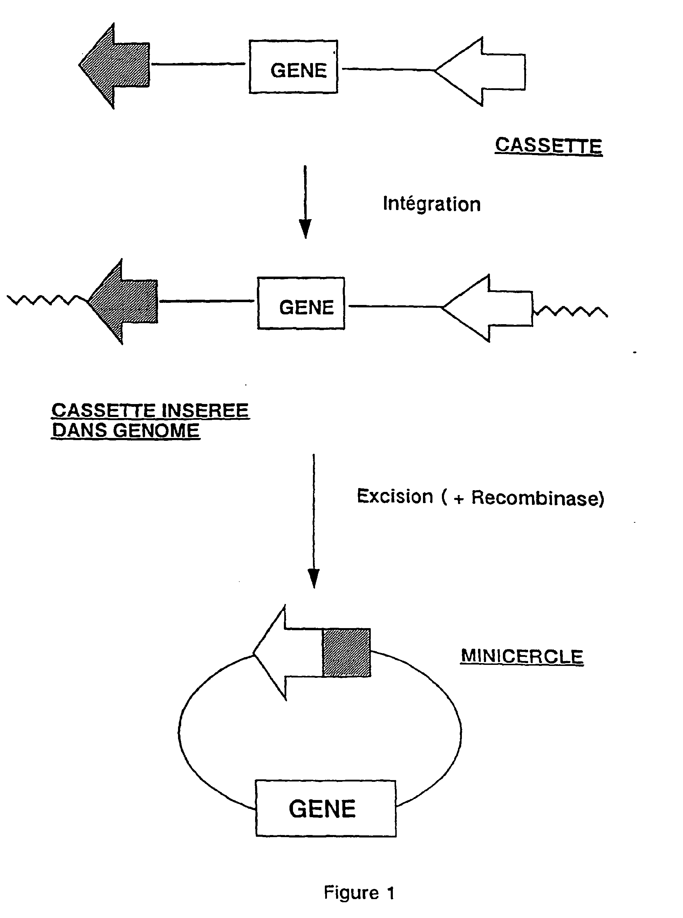

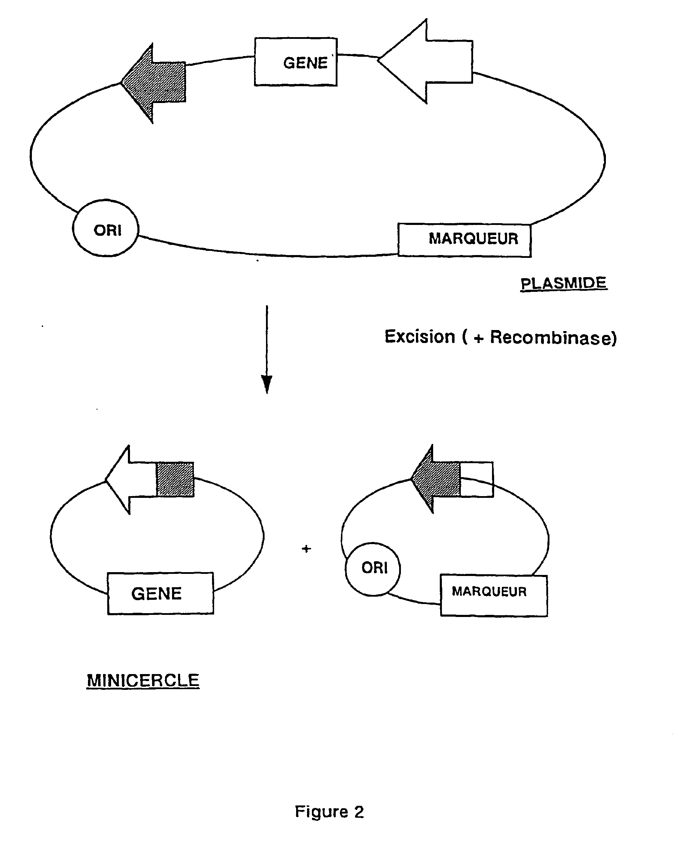

A cet égard, la présente invention fournit également des constructions génétiques particulières appropriées à la production des molécules d'ADN thérapeutiques définies ci-avant. Ces constructions génétiques, ou ADN recombinants selon l'invention comprennent notamment le ou les gènes d'intérêt encadré(s) par les deux séquences permettant la recombinaison site-spécifique positionnées en orientation directe. La position en orientation directe indique que les deux séquences suivent la même polarité 5'-3' dans l'ADN recombinant selon l'invention. Les constructions génétiques de l'invention peuvent être des fragments d'ADN double brin (cassettes) composées essentiellement des éléments indiqués ci-dessus. Ces cassettes sont utilisables pour la construction d'hôtes cellulaires ayant ces éléments intégrés dans leur génome (figure 1). Les constructions génétiques de l'invention peuvent également être des plasmides, c'est-à-dire toute molécule d'ADN, linéaire ou circulaire, capable de se répliquer dans une cellule hôte donnée, comportant le ou les gènes d'intérêt encadré(s) par les deux séquences permettant la recombinaison site-spécifique positionnées en orientation directe. D peut s'agir plus précisément d'un vecteur (tel qu'un vecteur de clonage et/ou d'expression), d'un phage, d'un virus, etc. Ces plasmides de l'invention peuvent être utilisés pour transformer tout hôte cellulaire compétent en vue de la production des minicercles par réplication du plasmide puis excision du minicercle (figure 2).

A cet égard, un autre objet de l'invention réside dans un ADN recombinant comprenant ADN recombinant comprenant une partie non thérapeutique et une partie thérapeutique ou cassette d'expression constituée d'un gène d'intérêt thérapeutique, vaccinal, agronomique ou vétérinaire, sous contrôle d'un promoteur et d'un terminateur transcriptionnels actifs dans les cellules mammifères encadrée par deux séquences permettant une recombinaison site spécifique, positionnées en orientation directe, la recombinaison par excision des parties non thérapeutiques permettant de générer la molécule selon l'invention génerer la molécule selon l'invention.

L'ADN recombinant selon l'invention est préférentiellement un plasmide comprenant au moins :
a) une origine de réplication et éventuellement un gène marqueur dans la partie non thérapeutique
b) deux séquences permettant une recombinaison site-spécifique positionnées en orientation directe, et.
c) placés entre lesdites séquences b), une cassette d'expression constituée d'un gène d'intrêt thérapeutique, vaccinal, agronomique ou vétérinaire, sous contrôle d'un promoteur et d'un terminateur transcriptionnels actifs dans les cellules mammifères.

Le système de recombinaison spécifique présent dans les constructions génétiques selon l'invention peut être de différentes origines. En particulier, les séquences spécifiques et les recombinases utilisées peuvent appartenir à différentes classes structurales, et notamment à la famille de l'intégrase du bactériophage λ ou à la famille de la résolvase du transposon Tn3.

Parmi les recombinases appartenant à la famille de l'intégrase du bactériophage λ, on peut citer notamment l'intégrase des phages lambda (Landy et al., Science 197 (1977) 1147), P22 et Φ80 (Leong et al., J. Biol. Chem. 260 (1985) 4468). HP1 de Haemophilus influenzae (Hauser et al., J. Biol. Chem. 267 (1992) 6859), l'intégrase Cre du phage P1, l'intégrase du plasmide pSAM2 (EP 350 341) ou encore la FLP recombinase du plasmide 2 µ. Lorsque les molécules d'ADN selon l'invention sont préparées par recombinaison au moyen d'un système site spécifique de la famille de l'intégrase du bactériophage lambda, les molécules d'ADN selon l'invention comprennent généralement en outre une séquence résultant de la recombinaison entre deux séquences d'attachement att du bactériophage ou plasmide correspondant.

Parmi les recombinases appartenant à la famille du transposon Tn3, on peut citer notamment la résolvase du transposon Tn3 ou des transposons , Tn21 et Tn522 (Stark et al., 1992) ; l'invertase Gin du bactériophage mu ou encore la résolvase de plasmides, telle que celle du fragment par de RP4 (Abert et al., Mol. Microbiol. 12 (1994) 131). Lorsque les molécules d'ADN selon l'invention sont préparées par recombinaison au moyen d'un système site spécifique de la famille du transposon Tn3, les molécules d'ADN selon l'invention comprennent généralement en outre une séquence résultant de la recombinaison entre deux séquences de reconnaissance de la résolvase du transposon considéré.

Selon un mode de réalisation particulier, dans les constructions génétiques de la présente invention, les séquences permettant la recombinaison site-spécifique sont dérivées d'un bactériophage. Plus préférentiellement, il s'agit des séquences d'attachement (séquences attP et attB) d'un bactériophage ou de séquences dérivées. Ces séquences sont capables de recombiner spécifiquement entre-elles en présence d'une recombinase désignée intégrase. Le terme séquence dérivée inclut les séquences obtenues par modification (s) des séquences d'attachement des bactériophages, conservant la capacité de recombiner spécifiquement en présence de la recombinase appropriée. Ainsi, il peut s'agir de fragments réduits de ces séquences ou au contraire étendus par addition d'autres séquences (sites de restriction, etc). Il peut également s'agir de variants obtenus par mutation(s), notamment par mutation(s) ponctuelle(s). On désigne selon l'invention par séquences attP et attB d'un bactériophage ou d'un plasmide les séquences du système de recombinaison spécifique dudit bactériophage ou plasmide, c'est-à-dire la séquence attP présente dans ledite phage ou plasmide et la séquence attB chromosomique correspondante.

A titre d'exemples préférés, on peut citer notamment les séquences d'attachement des phages lambda, P22, Φ80. P1, HP1 de Haemophilus influenzae ou encore du plasmide pSAM2. ou 2 µ. Ces séquences sont avantageusement choisies parmi tout ou partie des séquences SEQ ID n° 1, SEQ ID n° 2, SEQ ID n° 6, SEQ ID n° 7, SEQ ID n° 8. SEQ ID n° 9. SEQ ID n° 10. SEQ ID n° 11. SEQ ID n° 12, SEQ ID n° 13 et SEQ ID n° 14. Ces séquences comprennent notamment la région centrale homologue des séquences d'attachement de ces phages.

A cet égard, un plasmide selon la présente invention comprend :
(a) une origine de réplication bactérienne et éventuellement un gène marqueur dans la partie non-thérapeutique
(b) les séquences attP et attB d'un bactériophage sélectionné parmi les phages lambda, P22, Φ80, HP1, P1 ou du plasmide pSAM2 ou 2µ positionées en orientation directe, et,
(c) placés entre lesdites séquences b), une partie thérapeutique ou cassette d'expression constituée d'un géne d'intérêt thérapeutique, vaccinal, agronomique ou vétérinaire sous contrôle d'un promoteur et d'un terminateur transcriptionnels actifs dans les cellules mammifères.

Selon un mode particulièrement préféré de mise en oeuvre, il s'agit des séquences d'attachement (attP et attB) du phage lambda. Des plasmides portant ces séquences sont notamment les plasmides pXL2648, pXL2649 ou pXL2650. Lorsque ces plasmides sont mis, in vivo ou in vitro, en présence de l'intégrase du phage lambda, les séquences recombinent entre elles pour générer in vivo ou in vitro, par excision, un minicercle selon l'invention comprenant essentiellement les éléments (c), c'est-à-dire la partie thérapeutique (figure 2).

Toujours selon un mode particulier de réalisation de l'invention, permettant la recombinaison site-spécifique sont dérivées de la région IoxP du phage P1. Cène région se compose essentiellement de deux séquences repétées inversées capables de recombiner spécifiquement entre-elles en présence d'une protéine, désignée Cre (Stemberg et al., J. Mol. Biol. 150 (1971) 467). L'invention concerne donc un plasmide comprenant Plasmide
(a) une origine de réplication bactérienne et éventuellement un gène marqueur dans la partie non thérapeutique;
(b) les séquences répétées inversées du bactériophage P1 (région loxP) positionnées en orientation directe ; et,
(c) placés entre lesdites séquences (b), une partie thérapeutique ou cassette d'expression constituée d'un gène d'intérêt thérapeutique, vaccinal, agronomique ou vétérinaire sous contrôle d'un promoteur et d'un terminateur transcriptionnels actifs dans les cellules mammifères.

Selon un autre mode de réalisation particulier, dans les constructions génétiques de la présente invention, les séquences permettant la recombinaison site spécifique sont dérivées d'un transposon. Plus préférentiellement, il s'agit des séquences de reconnaissance de la résolvase d'un transposon ou de séquences dérivées. A titre d'exemples préférés, on peut citer notamment les séquences de reconnaissance des transposons Tn3, , Tn21 et Tn522. A titre d'exemple préféré, on peut citer la séquence SEQ ID n° 15 ou un dérivé de celle-ci (voir également Sherrat, P. 163-184, Mobile DNA. Ed. D. Berg and M. Howe. American Society for Microbiology, Washington D.C. 1989).

Selon la présente invention, les plasmides comprennent
a) une origine de réplication et éventuellement un gène marqueur dans la partie non thérapeutique,
b) deux séquences permettant une recombinaison site spécifique positionnées en orientation directe, et,
c) placés entre lesdites séquences b), une partie thérapeutique ou cassette d'expression constituée d'un ou plusieurs gènes d'intérêt thérapeutique, vaccinal, agronomique ou vétérinaire et d'une séquence capable de former une triple hélice par hybridation avec un oligonucléotide spécifique.

Selon une autre variante particulièrement avantageuse, les plasmides de l'invention comprennent en outre une séquence de résolution des multimeres. Il s'agit preferentiellement de la séquence mrs (multimer resolution system) du plasmide RK2. Plus preferentiellement, l'invention concerne un plasmide comprenant :
a) une origine de réplication et éventuellement un gène marqueur dans la partie non thérapeutique,
b) deux séquences permettant une recombinaison site spécifique positionnées en orientation directe, et,
c) placés entre lesdites séquences b), une partie thérapeutique ou cassette d'expression constituée d'un ou plusieurs gènes d'intérêt thérapeutique, vaccinal, agronomique ou vétérinaire et d'une séquence mrs issue du locus par de RK2, permettant de résoudre les multiméres.

Ce mode de réalisation est particulièrement avantageux. Ainsi, lorsque les plasmides pXL2649 ou pXL2650 sont mis en présence de l'intégrase du bactériophage in vivo les séquences recombinent pour générer le minicercle et le miniplasmide mais aussi des formes multimères ou topologiques de minicercle ou de miniplasmide. Il est particulièrement avantageux de pouvoir diminuer la concentration de ces formes pour augmenter la production et faciliter la purification de minicercle.

Les formes multimériques de plasmides sont connues de l'homme de l'art. Par exemple le fragment cer de ColE1 (Summers et coll. 1984 Cell 36 p1097) ou le site mrs du locus par de RK2 (L Ebert 1994 Mol. Microbiol. 2 p131) permettent la résolution de multimères de plasmides et participent à une stabilité accrue du plasmide. Mais alors que la résolution au site cer nécessite quatre protéines codées par le génome de E. coli (Colloms et coll. 1990 J. Bacteriol. 172 p6973), la résolution au site mrs ne nécessite que la protéine ParA dont le gène parA est cartographié sur le locus par de RK2. A ce titre, il semble avantageux d'utiliser tout ou une partie du locus par contenant parA et la séquence mrs. Par exemple, la séquence mrs peut être placée entre les séquences attB et attP du phage lambda et le gène parA être exprimé en trans ou en cis à partir de son propre promoteur ou d'un promoteur inductible. A cet egard. un plasmide particulier de l'invention comprend
a) une origine de réplication et éventuellement un gène marqueur dans la partie non thérapeutique,
b) deux séquences permettant une recombinaison site spécifique positionnées en orientation directe, et,
c) placés entre lesdites séquences b), une partie thérapeutique ou cassette d'expression constituée d'un ou plusieurs gènes d'intérêt thérapeutique, vaccinal, agronomique ou vétérinaire, d'une séquence mrs issue du locus par de RK2, permettant de résoudre les multiméres et une séquence capable de former une triple hélice par hybridation avec un oligonucléotide spécifique.

Un tel plasmide est notamment le plasmide pXL2960 décrit dans les exemples. Il peut être mis en oeuvre et permettre la production de minicercle sous forme monomérique uniquement.

Selon une autre variante avantageuse, les plasmides de l'invention comprennent
a) une origine de réplication et éventuellement un gène marqueur dans la partie non thérapeutique,
b) deux séquences permettant une recombinaison site spécifique intégrase dépendante positionnées en orientation directe et deux séquences permettant une recombinaison site spécifique résolvase dépendante positionnées à côté des deux premières et également en orientation directe, et,
c) placés entre lesdites séquences b), une partie thérapeutique ou cassette d'expression constituée d'un ou plusieurs gènes d'intérêt thérapeutique, vaccinal, agronomique ou vétérinaire et éventuellement d'une séquence capable de former une triple hélice par hybridation avec un oligonucléotide spécifique.
Les deux jeux de séquences de recombinaison site-spécifique sont d'une famille différente. Il s'agit avantageusement d'un premier jeu de séquences integrase-dépendantes et d'un second jeu de séquences par A-dépendantes. L'utilisation de deux jeux de séquences permet d'augmenter les rendements de production de minicercles, lorsque la première recombinaison site-spécifique est incomplète. Ainsi, lorsque les plasmides pXL2650 ou pXL2960 sont mis en présence de l'intégrase du bactériophage in vivo, les séquences recombinent pour générer le miniplasmide et le minicercle mais cette réaction n'est pas totale (il peut subsister 5 à 10 % de plasmide initial). L'introduction, à proximité de chacune des séquences att du phage lambda, d'une séquence mrs de RK2 permet d'augmenter la production de minicercles. Ainsi, après induction de l'intégrase du phage lambda et recombinaison Int-dépendante, les molécules non recombinées pourront être prise en charge par la protéine ParA de RK2 et pourront recombiner aux sites mrs. Inversement, après induction de la protéine ParA et recombinaison ParAdépendante, les molécules non recombinées pourront être prise en charge par l'intégrase du phage lambda et pourront recombiner aux sites att. De telles constructions permettent ainsi de produire du minicercle et des quantités négligeables de molécules non recombinées. Les séquences att, de même que les séquences mrs sont en orientation directe, et les gènes int et parA peuvent être induits simultanément ou successivement à partir du même promoteur inductible ou de deux promoteurs inductibles. Préférentiellement, il s'agit des séquences d'attachement attB et attP du phage lambda en orientation directe et de deux séquences mrs de RK2 en orientation directe.

Comme indiqué précédemment, un autre aspect de la présente invention réside dans un procédé de production de molécules d'ADN thérapeutiques définies ci-avant par excision, à partir d'un plasmide ou d'un chromosome, par recombinaison site-spécifique.

Un autre objet de la présente invention réside donc dans un procédé de production d'une molécule d'ADN (minicercle) telle que définie ci-avant selon lequel une culture de cellules hôtes contenant un ADN recombinant tel que défini ci-avant est mise en contact avec la recombinase permettant d'induire la recombinaison site-spécifique. Plus préférentiellement, la mise en contact est effectuée soit par transfection ou infection avec un plasmide ou un phage contenant le gène de la dite recombinase; soit par induction de l'expression d'un gène codant pour la dite recombinase, présent dans la cellule hôte. Comme indiqué ci-après, ce gène peut être présent dans la cellule hôte sous une forme intégrée au génome, sur un plasmide réplicatif ou encore sur le plasmide de l'invention, dans la partie non-thérapeutique.

Pour permettre la production des minicercles selon l'invention par recombinaison site-spécifique in vivo, la recombinase utilisée doit être introduite ou induite dans les cellules ou le milieu de culture au moment déterminé. Pour cela, différentes méthodes peuvent être utilisées. Selon une première méthode, on utilise une cellule hôte contenant le gène de la recombinase sous une forme permettant son expression régulée. Il peut notamment être introduit sous contrôle d'un promoteur ou d'un système de promoteurs inductibles, ou encore dans un système thermosensible. En particulier, le gène peut être présent dans un phage thermosensible, latent pendant la phase de croissance, et induit à température appropriée (exemple phage lysogène lambda Xis⁻ cI857). La cassette d'expression du gène de la recombinase peut être portée par un plasmide, un phage, ou même par le plasmide de l'invention, dans la région non-thérapeutique. Elle peut être intégrée au génome de la cellule hôte ou maintenue sous forme réplicative. Selon une autre méthode, la cassette d'expression du gène est portée par un plasmide ou un phage utilisé pour transfecter ou infecter la culture cellulaire après la phase de croissance. Dans ce cas, il n'est pas nécessaire que le gène soit sous une forme permettant son expression régulée. En particulier, tout promoteur constitutif peut être utilisé. La mise en contact avec la recombinase peut également être réalisée in vitro, sur un préparation de plasmide, par incubation directe avec la protéine.

Préférentiellement, on utilise dans le cadre de la présente invention une cellule hôte capable d'exprimer de manière régulée le gène de la recombinase. Ce mode de mise en oeuvre, dans lequel la recombinase est fournie directement par la cellule hôte après induction, est particulièrement avantageux. En effet, il suffit simplement de placer, au moment voulu, les cellules en culture dans les conditions d'expression du gène de la recombinase (température permissive pour gène thermosensible, ajout d'un inducteur pour promoteur régulable, etc) pour induire la recombinaison site-spécifique in vivo et ainsi l'excision du minicercle de l'invention. En outre, cette excision se fait avec des rendements particulièrement élevés puisque toutes les cellules en culture expriment la recombinase, ce qui n'est pas nécessairement le cas si une transfection ou une infection doivent être réalisées pour transférer le gène de la recombinase.

Selon un premier mode de réalisation, le procédé de l'invention comprend l'excision des molécules d'ADN thérapeutique par recombinaison site-spécifique à partir d'un plasmide. Ce mode de réalisation met en oeuvre les plasmides décrits ci-avant permettant, dans un premier temps, la réplication chez un hôte choisi puis, dans un second temps, l'excision des parties non-thérapeutiques du dit plasmide (notamment l'origine de réplication et le gène de résistance) par recombinaison site-spécifique, générant les molécules d'ADN circulaires de l'invention. Pour la mise en oeuvre du procédé, différents types de plasmides peuvent être utilisés et en particulier un vecteur, un phage ou un virus. Il s'agit préférentiellement d'un vecteur réplicatif.

Avantageusement, le procédé de l'invention comprend une étape préalable de transformation de cellules hôtes avec un plasmide tel que défini précédemment, puis de culture des cellules transformées, permettant d'obtenir des quantités appropriées de plasmide. L'excision par recombinaisons site-spécifique est alors réalisée par mise en contact avec la recombinase dans les conditions définies ci-avant (figure 2). Comme indiqué précédemment, dans ce mode de réalisation, la recombinaison site-spécifique peut être réalisée in vivo. (c'est-à-dire dans la cellule hôte) ou in vitro (c'est-à-dire sur une préparation de plasmide).

Selon un mode préféré, les molécules d'ADN de l'invention sont donc obtenues à partir d'un vecteur réplicatif, par excision de la partie non-thérapeutique portant notamment l'origine de réplication et le gène marqueur par recombinaison site-spécifique.

Selon un autre mode de réalisation, le procédé de l'invention comprend l'excision des molécules d'ADN à partir du génome de l'hôte cellulaire par recombinaison site-spécifique. Ce mode de réalisation repose plus particulièrement sur la construction d'hôtes cellulaires comprenant, inséré dans leur génome, une ou plusieurs copies d'une cassette comprenant le gène d'intérêt encadré par les séquences permettant la recombinaison (figure 1). Différentes techniques peuvent être utilisées pour l'insertion de la cassette de l'invention dans le génome de la cellule hôte. En particulier, l'insertion en plusieurs endroits distincts du génome peut être obtenue en utilisant des vecteurs intégratifs. A cet égard, différents systèmes de transposition tels que notamment le système miniMu ou les transposons défectifs tels que des dérivés de Tn10 par exemple peuvent être utilisés (Kleckner et al., Methods Enzymol. 204 (1991) 139; Groisman E., Methods Enzymol. 204 (1991) 180). L'insertion peut également être réalisée par recombinaison homologue, permettant d'intégrer dans le génome de la bactérie une cassette contenant deux séquences de recombinaison en orientation directe encadrant un ou plusieurs gènes d'intérêt. Ce processus peut en outre être reproduit autant de fois que désiré de manière à avoir un nombre de copies le plus important possible par cellule. Une autre technique consiste encore à utiliser un système d'amplification in vivo utilisant la recombinaison tel que décrit dans Labarre et al. (Labarre J., O. Reyes, Guyonvarch, and G. Leblon. 1993. Gene replacement, intégration, and amplification at the gdhA locus of Corynebacterium glutamicum. J. Bacteriol. 175:1001-107) de manière à passer d'une copie de la cassette à un nombre beaucoup plus important.

Une technique préférée consiste dans l'utilisation de miniMu. Des dérivés de miniMu sont construits à cet effet comprenant un marqueur de résistance, les fonctions nécessaires en cis à leur transposition et une cassette contenant deux séquences de recombinaison en orientation directe encadrant le ou les gènes d'intérêt. Ces miniMu sont avantageusement placées en plusieurs endroits du génome en utilisant un marqueur de résistance (la kanamycine, par exemple) permettant de sélectionner plusieurs copies par génome (Groisman E. précitée). Comme décrit ci-avant, la cellule hôte en question peut aussi exprimer de manière inductible une recombinase site spécifique conduisant à l'excision du fragment bordé par les séquences de recombinaison en orientation directe. Après excision les minicercles peuvent être purifiés par les techniques classiques.

Ce mode de mise en oeuvre du procédé de l'invention est particulièrement intéressant puisqu'il conduit à la génération d'un seul type de molécule plasmidique : le minicercle de l'invention. Les cellules ne contiennent en effet aucun autre plasmide episomal comme c'est le cas lors de la production à partir d'un plasmide (figures 1 et 2).

Un autre objet de l'invention réside également dans une cellule hôte modifiée comprenant, inséré dans son génome, une ou plusieurs copies d'un ADN recombinant tel que défini ci-avant.

L'invention concerne également toute cellule recombinante contenant un plasmide tel que défini ci-avant. Ces cellules sont obtenues par toute technique connue de l'homme du métier permettant l'introduction d'un ADN dans une cellule donnée. Il peut s'agir notamment de transformation, électroporation, conjugaison, fusion de protoplastes ou toute autre technique connue de l'homme de l'art. S'agissant de la transformation, différents protocoles ont été décrits dans l'art antérieur. En particulier, la transformation de cellules peut être réalisée en traitant les cellules entières en présence d'acétate de lithium et de polyéthylène glycol selon la technique décrite par Ito et al. (J. Bacteriol. 153 (1983) 163-168), ou en présence d'éthylène glycol et de diméthylsulfoxyde selon la technique de Durrens et al. (Curr. Genet. 18 (1990) 7). Un protocole alternatif a également été décrit dans la demande de brevet EP 361 991. S'agissant d'électroporation, elle peut être réalisée selon Becker et Guarentte (in : Methods in Enzymology Vol194 (1991) 182).

Le procédé selon l'invention peut être réalisé dans tout type d'hôte cellulaire. En particulier, il peut s'agir de bactéries ou de cellules eucaryotes (levures, cellules animales, cellules végétales) etc. Parmi les bactéries, on peut citer plus préférentiellement E.coli, B. subtilis, Streptomyces, Pseudomonas (P. putida, P. aeruginosa), Rhizobium meliloti, Agrobacterium tumefaciens, Staphylococcus aureus, Streptomyces pristinaespiralis, Enterococcus faecium ou Clostridium, etc. Parmi les bactéries, on préfère utiliser E. coli. Parmi les levures, on peut citer Kluyveromyces, Saccharomyces, Pichia, Hansenula, etc. Parmi les cellules animales de mammifères, on peut citer les cellules CHO, COS, NIH3T3. etc.

En fonction de l'hôte utilisé, le plasmide selon l'invention est adapté par l'homme du métier pour permettre sa réplication. En particulier, l'origine de réplication et le gène marqueur sont choisis en fonction de la cellule hôte selectionnée.

Le gène marqueur peut être un gène de résistance, notamment à un antibiotique (ampicilline, kamamycine, généticine, hygromycine, etc), ou tout gène conférant à la cellule une fonction qu'elle ne possède plus (par exemple un gène qui a été délété sur le chromosome ou rendu inactif), le gène sur le plasmide rétablissant cette fonction.

Dans un mode de réalisation particulier, le procédé de l'invention comprend une étape supplémentaire de purification du minicercle.

A cet égard, le minicercle peut être purifié par les techniques classiques de purification d'ADN plasmidique puisqu'il est superenroulé comme de l'ADN plasmidique. Ces techniques comprennent, entre autre, la purification sur gradient de densité en chlorure de césium, en présence de bromure d'éthidium, ou bien l'utilisation de colonnes d'échange d'anions (Maniatis et al., 1989). En outre si l'on estime que l'ADN plasmidique correspondant aux parties non-thérapeutiques (origine de réplication et marqueur de sélection notamment) est présent en quantité trop importante, il est également possible, après ou avant la purification, d'utiliser une ou plusieurs enzymes de restriction qui digéreront le plasmide et pas le minicercle, ce qui permet de les séparer par des techniques séparant l'ADN superenroulé de l'ADN linéaire, telles que un gradient de densité en chlorure de césium, en présence de bromure d'éthidium (Maniatis et al., 1989).

En outre, la présente invention décrit également un procédé amélioré pour la purification des minicercles. Ce procédé permet, en une seule étape, d'obtenir des minicercles de très grande pureté avec des rendements importants. Ce procédé amélioré est basé sur l'interaction entre une séquence double-brin présente sur le minicercle et un ligand spécifique. Le ligand peut être de nature diverse et notamment de nature protéique, chimique ou nucléique. Il s'agit préférentiellement d'un ligand de type acide nucléique, et notamment d'un oligonucléotide, éventuellement modifié chimiquement, capable de former par hybridation une triple-hélice avec la séquence spécifique présente sur la molécule d'ADN de l'invention. Il a en effet été montré que certains oligonucléotides étaient capables de former spécifiquement des triple-hélices avec des séquences d'ADN double-brin (Hélène et al., Biochim. Biophys. Acta 1049 (1990) 99; voir également FR94 15162 incorporée à la présente par référence).

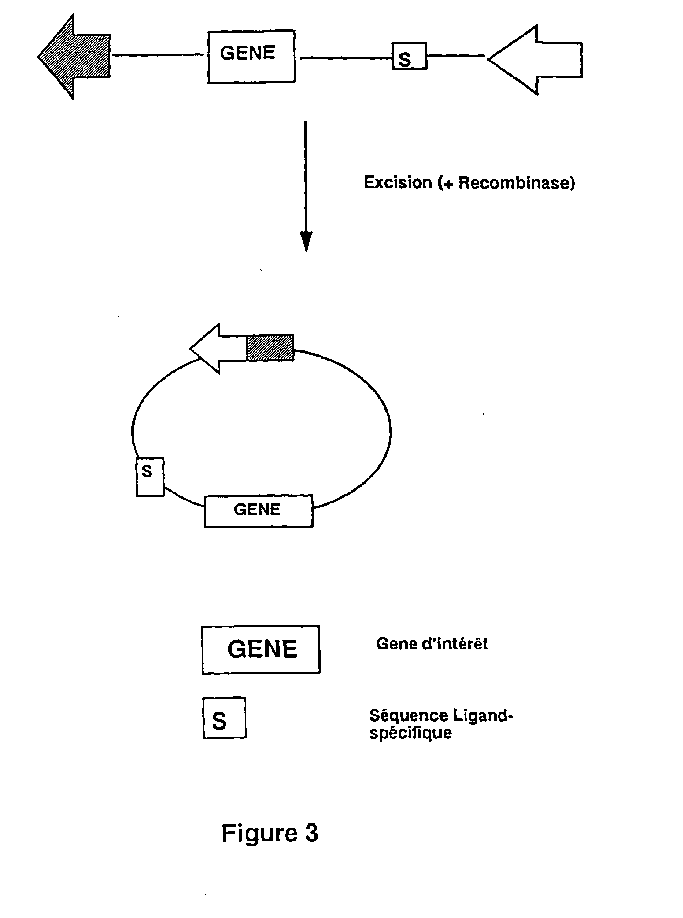

Dans une variante particulièrement avantageuse, les molécules d'ADN de l'invention contiennent donc en outre une séquence capable d'interagir spécifiquement avec un ligand (figure 3). De manière préférentielle, il s'agit d'une séquence capable de former, par hybridation, une triple-hélice avec un oligonucléotide spécifique. Cette séquence peut être positionnée en tout site de la molécule d'ADN de l'invention, dès lors qu'elle n'affecte pas la fonctionnalité du gène d'intérêt. Cette séquence est également présente dans les constructions génétiques de l'invention (plasmides, cassettes), dans la partie comportant le gène d'intérêt (voir notamment le plasmide pXL2650). Préférentiellement, la séquence spécifique présente sur la molécule d'ADN de l'invention comprend entre 5 et 30 paires de bases.

Les oligonucléotides utilisés pour la mise en oeuvre du procédé selon l'invention peuvent contenir les bases suivantes :
- thymidine (T), qui est capable de former des triplets avec les doublets AT de l'ADN double-brin (Rajagopal et al, Biochem 28 (1989) 7859);
- adénine (A), qui est capable de former des triplets avec les doublets AT de l'ADN double-brin;
- guanine (G), qui est capable de former des triplets avec les doublets G.C de l'ADN double-brin;
- cytosine protonée (C+), qui est capable de former des triplets avec les doublets G.C de l'ADN double-brin (Rajagopal et al précitée);

Préférentiellement, l'oligonucléotide utilisé comprend une séquence homopyrimidique contenant des cytosines et la séquence spécifique présente sur la molécule d'ADN est une séquence homopurique-homopyrimidique. La présence de cytosines permet d'avoir une triple hélice stable à pH acide, où les cytosines sont protonées, et déstabilisée à pH alcalin, où les cytosines sont neutralisées.

Pour permettre la formation d'une triple-hélice par hybridation, il est important que l'oligonucléotide et la séquence spécifique présente sur la molécule d'ADN de l'invention soient complémentaires. A cet égard, pour obtenir les meilleurs rendements et la meilleure sélectivité, on utilise dans le procédé de l'invention un oligonucléotide et une séquence spécifique parfaitement complémentaires. Il peut s'agir en particulier d'un oligonucléotide poly-CTT et d'une séquence spécifique poly-GAA. A titre d'exemple, on peut citer l'oligonucléotide de séquence GAGGCTTCTTCTTCTTCTTCTTCTT (SEQ ID n° 5), dans lequel les bases GAGG ne forment pas de triple hélice mais permettent d'espacer l'oligonucléotide du bras de couplage.

Il est entendu toutefois que certains mésappariements peuvent être tolérés, dès lors qu'ils ne conduisent pas à une perte trop grande d'affinité. L'oligonucléotide utilisé peut être naturel (composé de bases naturelles, non modifiées) ou modifié chimiquement. En particulier, l'oligonucléotide peut présenter avantageusement certaines modifications' chimiques permettant d'augmenter sa résistance ou sa protection vis à vis des nucléases, ou son affinité vis à vis de la séquence spécifique.

Ainsi, l'oligonucléotide peut être rendu plus résistant aux nucléases par modification du squelette (ex. methylphosphonates, phosphorothiates, phosphotriester, phosphoramidate, etc...). Un autre type de modification a plus particulièrement pour objet d'améliorer l'interaction et/ou l'affinité entre l'oligonucléotide et la séquence spécifique. En particulier, une modification tout à fait avantageuse selon l'invention consiste à méthyler les cytosines de l'oligonucléotide. L'oligonucléotide ainsi méthylé présente la propriété remarquable de former une triple hélice stable avec la séquence spécifique à pH neutre. Il permet donc de travailler à des pH plus élevés que les oligonucléotides de l'art antérieur, c'est-à-dire à des pH où les risques de dégradation de l'ADN plasmidique sont inférieurs.

La longueur de l'oligonucléotide utilisé dans le procédé de l'invention est d'au moins 3 bases, et de préférence, comprise entre 5 et 30. On utilise de manière avantageuse un oligonucléotide de longueur supérieure à 10 bases. La longueur peut être adaptée au cas par cas par l'homme du métier en fonction de la sélectivité et de la stabilité de l'interaction recherchées.

Les oligonucléotides selon l'invention peuvent être synthétisés par toute technique connue. En particulier, ils peuvent être préparés au moyen de synthétiseurs d'acides nucléiques. Toute autre méthode connue de l'homme du métier peut bien évidemment être utilisée.

Pour la mise en oeuvre du procédé de l'invention, le ligand spécifique (protéine, acide nucléique, etc) peut être greffé ou non sur un support. Différents types de supports peuvent être utilisés à cet effet, tels que notamment des supports de chromatographie fonctionnalisés, en vrac ou préconditionnés en colonne, des surfaces plastiques fonctionnalisées ou de billes de latex fonctionnalisées, magnétiques ou non. Il s'agit préférentiellement de supports de chromatographie. A titre d'exemple, les supports de chromatographie pouvant être utilisés sont l'agarose, l'acrylamide ou le Dextran ainsi que leurs dérivés (tels que Séphadex, Sépharose, Superose,...), les polymères tels que le poly(styrènedivinylbenzène), ou la silice greffée ou non greffée, par exemple. Les colonnes de chromatographie peuvent fonctionner en mode de diffusion ou de perfusion.

Pour permettre son couplage covalent sur le support, le ligand est généralement fonctionnalisé. S'agissant d'un oligonucléotide, il peut être modifié par exemple par un groupement terminal thiol, amine ou carboxyle, en position 5' ou 3'. En particulier, l'ajout d'un groupe thiol, amine ou carboxyle permet, par exemple, de coupler l'oligonucléotide sur un support portant des fonctions disulfure, maléimide, amine, carboxyle, ester, époxyde, bromure de cyanogène ou aldéhyde. Ces couplages se forment par établissement de liaisons disulfure, thioether, ester, amide ou amine entre l'oligonucléotide et le support. Toute autre méthode connue de l'homme du métier peut être utilisée, telle que des réactifs de couplage bifonctionnels, par exemple.

Par ailleurs, pour améliorer l'activité de l'oligonucléotide couplé, il peut être avantageux d'effectuer le couplage au moyen d'un "bras". L'utilisation d'un bras permet en effet de fixer l'oligonucléotide à une distance choisie du support permettant d'améliorer ses conditions d'interaction avec la molécule d'ADN de l'invention. Le bras est avantageusement constitué de bases nucléotidiques, n'interférant pas avec l'hybridation. Ainsi, le bras peut comprendre des bases puriques. A titre d'exemple, le bras peut comprendre la séquence GAGG.

Les molécules d'ADN selon l'invention peuvent être utilisées dans toute application de vaccination ou de thérapie génique et cellulaire, pour le transfert d'un gène à un organisme, un tissu ou une cellule donnée. En particulier, elles peuvent être utilisées pour une administration directe in vivo, ou pour la modification de cellules in vitro ou ex vivo, en vue de leur implantation à un patient. A cet égard, les molécules selon l'invention peuvent être utilisées telles quelles (sous forme d'ADN nu), ou en association avec différents vecteurs chimiques et/ou biochimiques, synthétiques ou naturels. Il peut s'agir notamment de cations (phosphate de calcium, DEAE-dextran,...) qui agissent en formant des précipités avec l'ADN, lesquels peuvent être "phagocytés" par les cellules. Il peut également s'agir de liposomes dans lesquels la molécule d'ADN est incorporée et qui fusionnent avec la membrane plasmique. Les vecteurs synthétiques de transfert de gènes sont généralement des lipides ou polymères cationiques qui complexent l'ADN et forment avec lui une particule portant des charges positives en surface. Ces particules sont capables d'interagir avec les charges négatives de la membrane cellulaire, puis de franchir celle-ci. On peut citer comme exemples de tels vecteurs le DOGS (Transfectam^{™}) ou le DOTMA (Lipofectin^{™}). Des protéines chimères ont aussi été développées : elles sont constituées d'une partie polycationique qui condense l'ADN, liée à un ligand qui se fixe sur un récepteur membranaire et entraîne le complexe dans les cellules par endocytose. Les molécules d'ADN selon l'invention peuvent également être utilisées pour le transfert de gènes dans des cellules par des techniques physiques de transfection telles que le bombardement, l'électroporation, etc. En outre, préalablement à leur utilisation thérapeutique, les molécules de l'invention peuvent éventuellement être linéarisées par exemple par coupure enzymatique.

A cet égard, un autre objet de la présente invention concerne toute composition pharmaceutique comprenant une molécule d'ADN au moins telle que définie ci-avant. Cette molécule peut être nue ou associée à un vecteur chimique et/ou biochimique de transfection. Les compositions pharmaceutiques selon l'invention peuvent être formulées en vue d'administrations par voie topique, orale, parentérale, intranasale, intraveineuse, intramusculaire, sous-cutanée, intraoculaire, transdermique, etc. De préférence, la molécule d'ADN est utilisée sous une forme injectable ou en application. Elle peut être mélangée à tout véhicule pharmaceutiquement acceptable pour une formulation injectable, notamment pour une injection directe au niveau du site à traiter. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Il peut s'agir notamment de tampons Tris ou PBS dilués dans du glucose ou du chlorure de sodium. Une injection directe de l'acide nucléique dans la région atteinte du patient est intéressante car elle permet de concentrer l'effet thérapeutique au niveau des tissus affectés. Les doses d'acide nucléique utilisées peuvent être adaptées en fonction de différents paramètres, et notamment en fonction du gène, du vecteur, du mode d'administration utilisé, de la pathologie concernée ou encore de la durée du traitement recherchée.

Les molécules d'ADN de l'invention peuvent comporter un ou plusieurs gènes d'intérêt, c'est-à-dire un ou plusieurs acides nucléiques (ADNc, ADNg, ADN synthétique ou semi-synthétique, etc) dont la transcription et éventuellement la traduction dans la cellule cible génèrent des produits ayant un intérêt thérapeutique, vaccinal, agronomique ou vétérinaire.

Parmi les gènes d'intérêt thérapeutique, on peut citer plus particulièrement les gènes codant pour des enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, BFGF, NT3, NT5, etc; les apolipoprotéines : ApoAI, ApoAIV, ApoE, etc (FR 93 05125), la dystrophine ou une minidystrophine (FR 9111947), les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, etc, les gènes suicides : Thymidine kinase, cytosine désaminase, etc; ou encore tout ou partie d'une immunoglobuline naturelle ou artificielle (Fab, ScFv, etc), un ARN ligand (WO91/19813) etc. Le gène thérapeutique peut également être un gène ou une séquence antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine, selon la technique décrite dans le brevet EP 140 308.

Le gène d'intérêt peut aussi être un gène vaccinant, c'est-à-dire un gène codant pour un peptide antigénique, capable de générer chez l'homme ou l'animal une réponse immunitaire, en vue de la réalisation de vaccins. Il peut s'agir notamment de peptides antigéniques spécifiques du virus d'epstein barr, du virus HIV, du virus de l'hépatite B (EP 185 573), du virus de la pseudo-rage, ou encore spécifiques de tumeurs (EP 259 212).

Généralement, dans les plasmides et molécules de l'invention, le gène d'intérêt thérapeutique, vaccinal, agronomique ou vétérinaire contient également une région promotrice de la transcription fonctionnelle dans la cellule ou l'organisme cible (i.e. les mammifères), ainsi qu'une région située en 3', et qui spécifie un signal de fin transcriptionnelle et un site de polyadénylation (cassette d'expression). Concernant la région promotrice, il peut s'agir d'une région promotrice naturellement responsable de l'expression du gène considéré lorsque celle-ci est susceptible de fonctionner dans la cellule ou l'organisme concernés. Il peut également s'agir de régions d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule cible. Parmi les promoteurs eucaryotes, on peut utiliser tout promoteur ou séquence dérivée stimulant ou réprimant la transcription d'un gène de façon spécifique ou non, inductible ou non, forte ou faible. Il peut s'agir en particulier de promoteurs ubiquitaires (promoteur des gènes HPRT, PGK, α-actine, tubuline, etc), de promoteurs des filaments intermédiaires (promoteur des gènes GFAP, desmine, vimentine, neurofilaments, kératine, etc), de promoteurs de gènes thérapeutiques (par exemple le promoteur des gènes MDR, CFTR, Facteur VIII, ApoAI, etc), de promoteurs spécifiques de tissus (promoteur du gène pyruvate kinase, villine, protéine intestinale de liaison des acides gras, α-actine du muscle lisse, etc) ou encore de promoteurs répondant à un stimulus (récepteur des hormones stéroïdes, récepteur de l'acide rétinoïque, etc). De même, il peut s'agir de séquences promotrices issues du génome d'un virus, tel que par exemple les promoteurs des gènes E1A et MLP d'adénovirus, le promoteur précoce du CMV, ou encore le promoteur du LTR du RSV, etc. En outre, ces régions promotrices peuvent être modifiées par addition de séquences d'activation, de régulation, ou permettant une expression tissu-spécifique ou majoritaire.

Par ailleurs, le gène d'intérêt peut également comporter une séquence signal dirigeant le produit synthétisé dans les voies de sécrétion de la cellule cible. Cette séquence signal peut être la séquence signal naturelle du produit synthétisé, mais il peut également s'agir de toute autre séquence signal fonctionnelle, ou d'une séquence signal artificielle.

Selon le gène d'intérêt, les molécules d'ADN de l'invention peuvent être utilisées pour le traitement ou la prévention de nombreuses pathologies, incluant les maladies génétiques (dystrophie, fibrose cystique, etc), les maladies neurodégénératives (alzheimer, parkinson, ALS, etc), les cancers, les pathologies liées aux désordres de la coagulation ou aux dyslipoprotéinémies, les pathologies liées aux infections virales (hépatites, SIDA, etc), ou dans les domaines agronomique et vétérinaire, etc.

La présente invention sera plus complètement décrite à l'aide des exemples qui suivent, qui doivent être considérés comme illustratifs et non limitatifs.

### Légende des Figures

Figure 1 : Production d'un minicercle à partir d'une cassette intégrée dans le génome.
Figure 2 : Production d'un minicercle à partir d'un plasmide.
Figure 3 : Production d'un minicercle contenant une séquence spécifique d'un ligand.
Figure 4 : Construction de pXL2649. Ori : Origine de réplication; Kan^{r} : Gène marqueur conférant la résistance à la kanamycine; Amp^{r} : Gène marqueur conférant la résistance à l'ampicilline; galK : Gène de la galactosidase de E. Coli; Plac : Promoteur de l'opéron lactose.
Figure 5 : Activité luciférase obtenue après transfection de fibroblastes murins NIH3T3 par le plasmide pXL2650, le minicercle généré à partir du plasmide pXL2650 et le PGL2-Control (Promega Biotech). La transfection a été réalisée dans les conditions suivantes: 0,5 mg d'ADN par puits, 50000 cellules par puits. Le lipofectant utilisé est le RPR 115335. Le résultat est rapporté en RLU par microgramme de protéines en fonction du rapport de charge lipofectant/ ADN.

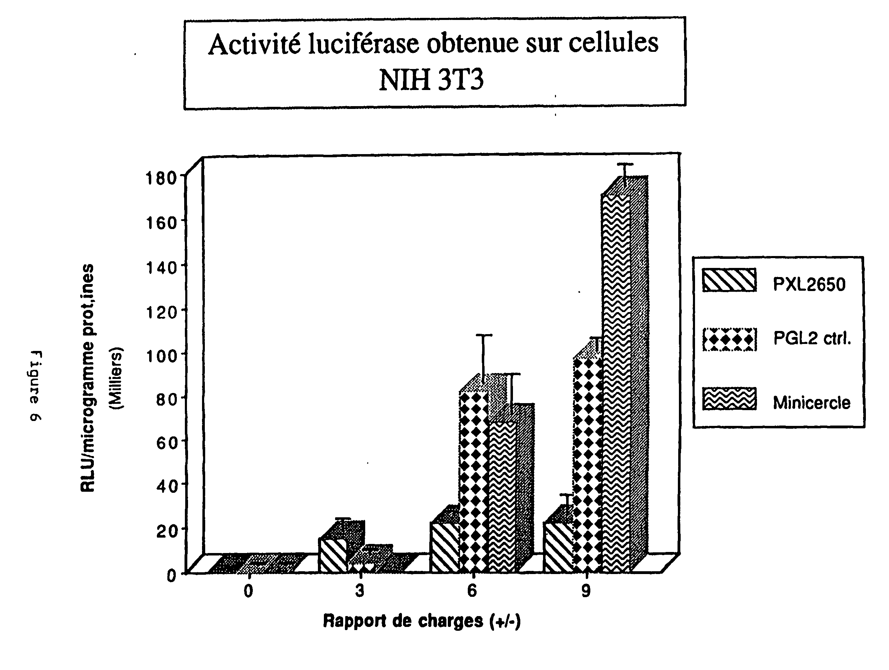

Figure 6: Construction du plasmide pXL2793. Ce plasmide génère après recombinaison un minicercle comportant une séquence synthétique homopurique-homopyrimidique et la casette luciférase du pXL2727.
Figure 7 : Le puits 1 correspond à la digestion de la fraction éluée après purification par colonne triple hélice par SalI. Le puits 2 correspond à la digestion de la fraction éluée après purification par colonne triple hélice par XmnI. Le puits 3 correspond à la fraction éluée après purification par colonne triple hélice, non digérée. Le puits 4 correspond au plasmide pXL2793 non induit, non digéré. Les puits 5 et 6 correspondent respectivement au marqueur de taille d'ADN linéaire et d'ADN superenroulé.
Figure 8. Description schématique de la construction du plasmide pXL2776.
Figure 9. Description schématique des constructions des plasmides pXL2777 et pXL2960.
Figure 10. Action de l'intégrase du bactériophage 1 chez E. coli sur les plasmides pXL2777 et pXL2960. M : marqueur de poids moléculaire 1 kb d'ADN linéaire, ou d'ADN surenroulé. N.I. : non induit. I : induit. N.D. : non digéré.

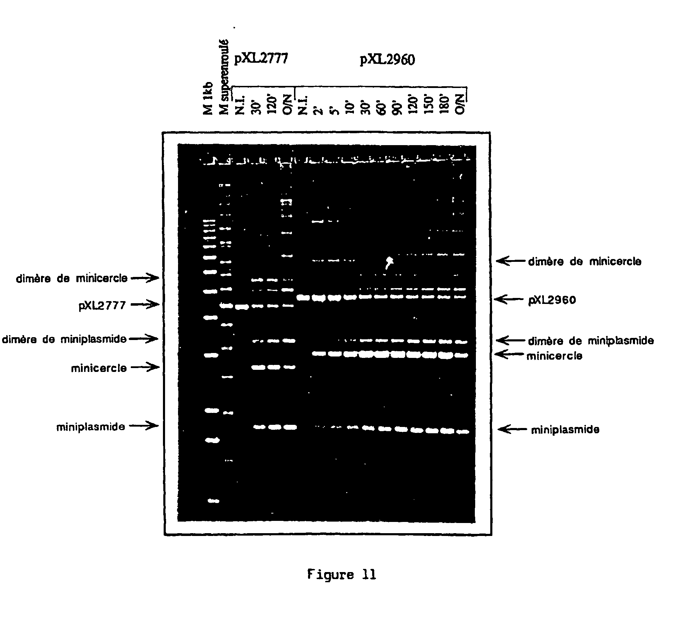

Figure 11. Cinétique de recombinaison de l'intégrase du bactériophage 1 chez E. coli sur les plasmides pXL2777 et pXL2960. 2': 2 minutes. O/N: 14 heures. M : marqueur de poids moléculaire 1 kb d'ADN linéaire, ou d'ADN surenroulé. N.I. : non induit. I : induit. N.D. : non digéré.

### Techniques générales de clonage et de biologie moléculaire

Les méthodes classiques de biologie moléculaire telles que la centrifugation d'ADN plasmidique en gradient de chlorure de césium-bromure d'éthidium, les digestions par des enzymes de restriction, l'électrophorèse sur gel, l'électroélution des fragments d'ADN à partir de gels d'agarose, la transformation dans E. coli, la précipitation des acides nucléiques etc, sont décrites dans la littérature (Maniatis et al., 1989, Ausubel et al., 1987). Les séquences nucléotidiques ont été déterminées par la méthode de terminaison de chaînes en suivant le protocole déjà présenté (Ausubel et al., 1987).

Les enzymes de restriction ont été fournies par New-England Biolabs (Biolabs), Bethesda Research Laboratories (BRL) ou Amersham Ltd (Amersham).

Pour les ligatures, les fragments d'ADN sont séparés selon leur taille sur des gels d'agarose à 0,7 % ou d'acrylamide à 8 %, purifiés par électrophorèse puis électroélution, extraits au phénol, précipités à l'éthanol puis incubés dans un tampon Tris-HCl pH 7.4 50 mM, MgCl2 10 mM, DIT 10 mM, ATP 2 mM, en présence d'ADN ligase du phage T4 (Biolabs). Les oligonucléotides sont synthétisés en utilisant la chimie des phosphoramidites protégés en b par un groupement cyanoéthyl (Sinha et al., 1984, Giles 1985) avec le synthétiseur automatique d'ADN Biosearch 8600 en utilisant les recommandations du fabricant.

Les ADN ligaturés sont utilisés pour transformer la souche rendue compétente †: E. coli MC1060 '[(lacIQPZYA)X74, galU, galK, strA^{r}, hsdR] (Casadaban et al., 1983) ; HB101 [hsdS20, supE44, recA13, ara-14, proA2, lacY1, galK2, rpsL20, xyl-5, mtl-1, λ⁻, F-] (Maniatis et al., 1989) et DH5α [endA1 hsdR17 supE44 thi-1 recA1 gyrA96 relA1 λ⁻ Φ80 dlacZΔM15] pour les plasmides.

Les milieux de culture LB et 2XTY sont utilisés pour la partie bactériologique (Maniatis et al., 1989).

Les ADN plasmidiques sont purifiés suivant la technique de lyse alcaline (Maniatis et al., 1989).

Définition des termes employés et abréviations.

ADN recombinant : ensemble de techniques qui permettent soit d'associer au sein du même microorganisme des séquences d'ADN qui ne le sont pas naturellement, soit de mutagéniser spécifiquement un fragment d'ADN.
ATP : adénosine 5'-triphosphate
BSA : sérum albumine bovine
PBS : tampon phosphate 10 mM, NaCl 150 mM, pH 7,4
dNTP : 2'-désoxyribonucléosides 5'-triphosphates
DTT : dithiothréitol
kb : kilobases
pb : paires de bases

### Exemple 1 : Construction d'un plasmide portant les séquences attP et attB, du bactériophage , en orientations directes répétées .

Le plasmide pNH16a a servi de matériel de départ dans la mesure ou il contient déjà un fragment de bactériophage λ portant la séquence attP (Hasan et Szybalski, 1987). Ce plasmide a été digéré par EcoRI. Des oligonucléotides qui contiennent la séquence attB (Landy, 1989) ont été synthétisés. Ils ont la séquence suivante
Oligonucléotide 5476 (SEQ ID n°1)
5'-AATTGTGAAGCCTGCTTTTTTATACTAACTTGAGCGG-3'
Oligonucléotide 5477 (SEQ ID n°2)
5'-AATTCCGCTCAAGTTAGTATAAAAAAGCAGGCTTCAC-3'

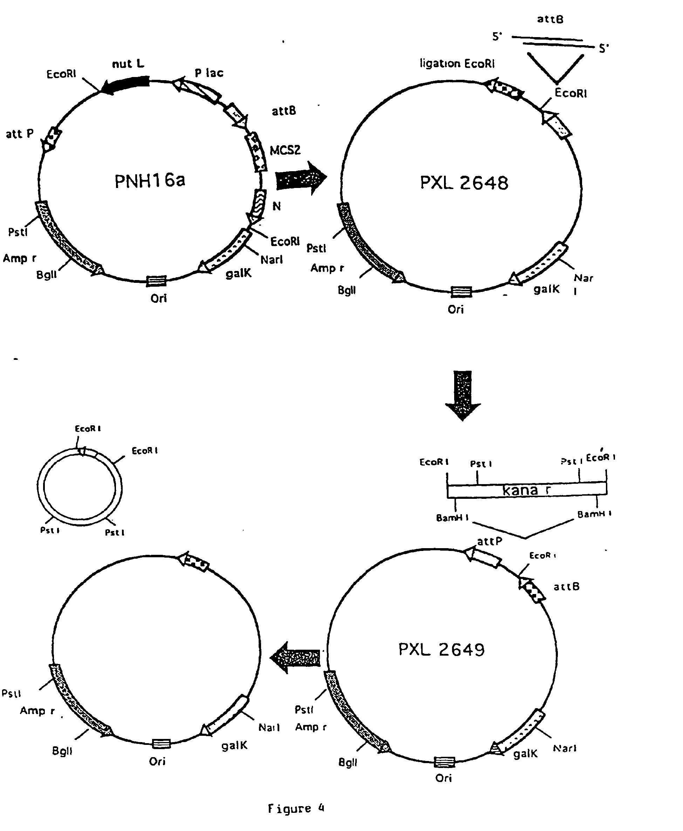

Us ont été hybridés pour reconstituer la séquence attB puis ligaturés au site EcoRI du fragment EcoRI de 4.2 kb du pNH16a (Hasan et Szybalski, 1987). Après transformation de DH5α, un clone recombinant a été conservé. Le plasmide ainsi construit a été nommé pXL2648 (voir Figure 4). Ce plasmide contient les séquences attP et attB du bactériophage en orientation directe. Sous l'action de l'intégrase du bactériophage (protéine Int) il doit y avoir excision des séquences comprises entre les deux sites att. Ceci conduit à séparer ce qui est inséré entre les deux séquences att, de l'origine de réplication et du marqueur de résistance du plasmide, positionnés à l'extérieur.

### Exemple 2 : Obtention in vivo chez E. coli d'un minicercle.

Une cassette de résistance à la kanamycine a été clonée au site EcoRI du plasmide pXL2648 (Figure 4). Cette cassette provient du plasmide pUC4KIXX (Pharmacia Biotech.). Pour ce faire, 10 g du plasmide pUC4KIXX ont été digérés par EcoRI, puis séparés par électrophorèse sur gel d'agarose; le fragment de 1.6 kb contenant le marqueur de résistance à la kanamycine a été purifié par électroélution; il a ensuite été ligaturé au plasmide pXL2648 linéarisé par EcoRI. Les clones recombinants ont été sélectionnés après transformation dans E. coli DH5α et sélection pour la résistance à la kanamycine. Le profil de restriction attendu a été observé sur un clone; ce clone plasmidique a été nommé pXL2649 (Figure 4). Ce plasmide a été introduit par transformation dans deux souches d'E. coli :
D1210 [hsdS20, supE44, recA13, ara-14, proA2, lacY1, aglK2, rpsL20, xyl-5, mtl-1, λ⁻, F-, lacIq] (Sadler et al., 1980)
D1210HP, qui correspond à DH1210 lysogénisée par le phage xis⁻ (Xis⁻ Kil⁻) cI857 (Podjaska et al., 1985).

Les transformants ont été sélectionnés à 30°C sur milieu 2XTY avec de la kanamycine (50 mg/l). Après réisolernent sur milieu sélectif, les souches ont été inoculées dans 5 ml de milieu L supplémenté avec de la kanamycine (50 mg/l). Après 16 hr d'incubation à 30°C avec agitation (5 cm d'amplitude rotative) les cultures ont été diluées au 1/100ème dans 100 ml du même milieu. Ces cultures ont été incubées dans les mêmes conditions jusqu'à atteindre une DO₆₁₀ de 0.3. A ce moment, la moitié de la culture a été prélevée puis incubée 10 mn à 42°C pour induire le cycle lytique du phage , donc l'expression de l'intégrase. Après cette incubation les cultures ont été à nouveau transférées à 30°C puis incubées 1 hr dans ces conditions. Ensuite, les cultures ont été arrêtées et des minipréparations d'ADN plasmidiques ont été réalisées. Quelles que soient les conditions, dans la souche D1210, le profil d'électrophorèse en gel d'agarose de l'ADN plasmidique non digéré du plasmide pXL2649 est inchangé ainsi que dans la souche D1210HP n'ayant pas été induite thermiquement. Au contraire dans D1210HP ayant été incubée 10 mn à 42 °C puis cultivée 1 heure à 30°C, on constate qu'il n'y a non plus un plasmide, mais deux molécules circulaires d'ADN : une de bas poids moléculaire, migrant le plus rapidement et contenant un site EcoRI; et une de plus haut poids moléculaire, contenant un site unique BglI, comme attendu. Il y a donc bien eu excision des séquences présentes entre les deux séquences att, et génération d'un minicercle dénué de toute origine de réplication. Cet ADN, circulaire, superenroulé, ne portant pas d'origine de réplication est désigné minicercle. Cette appelation rend en effet mieux compte du caractère circulaire de la molécule. Le plasmide pXL2649 de départ est présent, mais il représente environ 10 % du plasmide ayant excisé les séquences bordées par att.

Le minicercle peut ensuite être purifié par les techniques classiques de purification d'ADN plasmidique puisqu'il est superenroulé comme de l'ADN plasmidique. Ces techniques comprennent, entre autre, la purification sur gradient de densité en chlorure de césium, en présence de bromure d'éthidium, ou bien l'utilisation de colonnes d'échange d'anions (Maniatis et al., 1989). En outre si l'on estime que l'ADN plasmidique correspondant à l'origine de réplication et au marqueur de sélection est présent en quantité trop importante, il est toujours possible après purification d'utiliser une ou plusieurs enzymes de restriction qui digéreront le plasmide et pas le minicercle, ce qui permet de les séparer par des techniques séparant l'ADN superenroulé de l'ADN linéaire, telles qu'en gradient de densité en chlorure de césium, en présence de bromure d'éthidium (Maniatis et al., 1989).

### Exemple 3 : Obtention d'un minicercle contenant une cassette d'expression de la luciférase.

Afin de tester l'utilisation in vivo de ces minicercles, un gène reporter avec les séquences nécessaires à son expression a été cloné dans le plasmide pXL2649 (cf exemple 2). Il s'agit plus particulièrement d'une cassette BglII-BamHI de 3150 pb issue de pGL2-Control (Promega Biotech.). Cette cassette contient le promoteur précoce de SV40, l'enhanceur du promoteur précoce de SV40, le gène de la luciférase de Photinus pyralis et un site de polyadénylation dérivé de SV40. Le fragment BglII-BamHI de 3150 bp a été cloné au site BamHI de pXL2649 digéré par BamHI de manière à remplacer la cassette de résistance à la kanamycine par la cassette d'expression de la luciférase de pGL2-control. Le plasmide ainsi construit a été appelé pXL2650. Dans ce plasmide les sites attP et *att*B bordent la cassette d'expression de la luciférase. La recombinaison site spécifique permet d'exciser uniquement les séquences nécessaires à l'expression de la luciférase ainsi que le gène de la luciférase. Celle-ci peut être réalisée exactement comme décrit à l'exemple 2. Un minicercle tel que le plasmide pXL2650 peut être ensuite utilisé dans des expériences de transfection in vivo ou in vitro.

Une culture de 1 litre de la souche D1210HP pXL2650, en milieu 2XTY supplémenté avec de l'ampicilline (50 mg/ml) a été réalisée à 30°C. A une DO₆₁₀ égale à 0.3, la culture a été transférée à 42°C pendant 20 mn, puis remise 20 mn à 30°C. L'ADN épisomal a été préparé par la technique du lysat clair (Maniatis et al., 1989) suivie d'un gradient de densité en chlorure de césium supplémenté avec du bromure d'éthidium (Maniatis et al., 1989), puis d'une extraction du bromure d'éthidium à l'isopropanol et d'une dialyse. Cet ADN a montré contenir le minicercle. 100 g de cette préparation ont été digérés par PstI, puis l'hydrolysat a été soumis à un gradient de densité en chlorure de césium supplémenté avec du bromure d'éthidium (Maniatis et al., 1989). Un resultat identique est obtenu lorsque la préparation est digérée conjointement par AlwNI et XmnI. La forme superenroulée a été récupérée et après élimination du bromure d'éthidium (Maniatis et al.), elle s'est avérée ne correspondre qu'au minicercle, dépourvu d'origine de réplication et de tout gène marqueur. Cette préparation de minicercle peut être utilisée pour des expériences de transfection in vitro et in vivo.

### Exemple 4 : Transfection in vitro de cellules de mammifères et plus particulièrement de cellules humaines par un minicercle.

L'ADN minicercle contenant le gène de la luciférase de Photinus pyralis tel que décrit à l'exemple 3, c'est à dire correspondant au minicercle généré à partir du plasmide pXL2650, est dilué dans du NaCl 150 mM et mélangé avec un transfectant. On peut utiliser divers transfectants commerciaux tels que le dioctadécylamidoglycylspermine (DOGS, Transfectam^{™}, Promega), la Lipofectin^{™} (Gibco-BRL), etc, dans différents rapports de charges positives/négatives. A titre illustratif, l'agent transfectant a été utilisé dans des rapports de charges supérieurs ou égaux à 3. Le mélange est vortexé, laissé 10 minutes à température ambiante, dilué dans du milieu de culture dépourvu de sérum de veau foetal, puis ajouté aux cellules, à raison de 2 µg d'ADN par puits de culture. Les cellules utilisées sont des Caco-2, dérivées d'un adénocarcinome du côlon humain, cultivées selon un protocole décrit (Wils et al., 1994) et ensemencées la veille de l'expérience dans des plaques de culture à 48 puits, à raison de 50.000 cellules/puits. Après deux heures à 37°C, on ajoute 10% v/v de sérum de veau foetal et les cellules sont incubées 24 heures à 37°C en présence de 5% de CO₂. Les cellules sont lavées deux fois au PBS et l'activité luciférase est mesurée selon le protocole décrit (tel que le kit Proméga). On peut utiliser d'autres lignées (fibroblastes, lymphocytes,...) provenant de différentes espèces, ou bien des cellules prélevées chez un individu (fibroblastes, kératinocytes, lymphocytes,...) et qui lui seront réinjectées après transfection.

### Exemple 5 : Transfection in vitro de cellules NIH 3T3

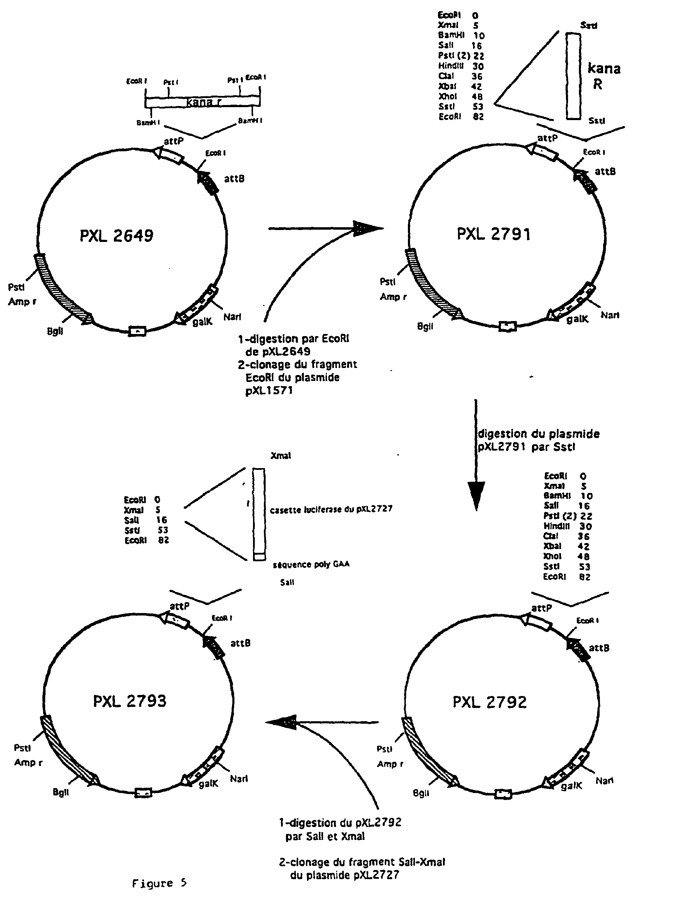

L'ADN minicercle contenant le gène de la luciférase de Photinus pyralis, tel que décrit à l'exemple 3, c'est à dire correspondant au minicercle généré à partir du plasmide pXL2650 a été transfecté in vitro dans des cellules de mammifères; le pXL2650 et le PGL2-Control (Promega Biotech.) qui comportent la même casette d'expression ont été utilisés comme contrôle. Les cellules utilisées sont des fibroblastes murins NIH 3T3, ensemencées la veille de l'expérience dans des plaques de culture à 24 puits, à raison de 50.000 cellules par puits. Le plasmide est dilué dans du NaCl 150 mM et mélangé avec le lipofectant RPR115335. Cependant on peut utiliser divers autres agents commerciaux tels que le dioctadécylamidoglycyl spermine (DOGS, Transfectam^{™}, Promega) (Demeneix et al. Int. J. Dev. Biol. 35 (1991) 481), la Lipofectin^{™} (Gibco-BRL) (Fegner et al. Proc, Natl. Acad. Sci. USA 84 (1987) 7413), etc. On utilise un rapport de charges positives du lipofectant/charges négatives de l'ADN égal ou supérieur à 3. Le mélange est vortexé, laissé dix minutes à température ambiante, dilué dans du milieu dépourvu de sérum de veau foetal, puis ajouté aux cellules, à raison de 0,5 mg d'ADN par puits de culture. Après deux heures à 37°C, on ajoute 10 % volume à volume de sérum de veau foetal et les cellules sont incubées 48 heures à 37°C en présence de 5 % de CO₂. Les cellules sont lavées deux fois avec du PBS et l'activité luciférase est mesurée selon le protocole décrit (kit Promega, Promega Corp. Madison, WI), sur un luminomètre Lumat LB9501 (EG et G Berthold, Evry). Les résultats de transfection correspondant aux conditions qui viennent d'être énoncées sont présentés figure 5. Ils montrent sans ambiguité que le minicercle présente les mêmes propriétés de transfection que des plasmides possédant une origine de réplication. Ainsi ces minicercles pourraient être utiliser indifféremment de plasmides standards dans des applications de thérapie génique.

### Exemple 6 : Purification d'un minicercle par affinité en utilisant une interaction hélice-triple.

Cet exemple décrit une méthode de purification d'un minicercle selon l'invention à partir d'un mélange comportant la forme plasmidique l'ayant excisé, par des interactions de type triple hélice qui vont se faire avec une séquence d'ADN synthétique portée par le minicercle à purifier. Cet exemple démontre comment la technologie de purification par formation d'une triple hélice peut être utilisée pour séparer un minicercle d'une forme plasmidique l'ayant excisé.

### 6-1. Obtention d'un minicercle comportant une séquence synthétique homopurique-homopyrimidique.

### 6-1.1. Insertion d'une séquence homopurique- homopyrimidique dans le plasmide pXL2650

Le plasmide pXL2650 possède un site unique BamHI juste après la cassette contenant le gène de la luciférase de Photinus pyralis. Ce site unique a été utilisé pour cloner les deux oligonucléotides suivants :
4957 (SEQ ID n°3)
5'-GATCCGAAGAAGAAGAAGAAGAAGAAGAAGAAGAAGAAGAAGA AGAAGAAGAAGAAC-3'
4958 (SEQ ID n°4)
5'-GATCGTTCTTCTTCTTCTTCTTCTTCTTCTTCTTCTTCTTCTTCTTC TTCTTCTTCG-3'

Ces oligonucléotides, une fois hybridés et clonés sur le plasmide pXL2650 apportent une séquence homopurique-homopyrimidique (GAA)₁₇, comme décrit ci-dessus.

Pour réaliser ce clonage les olignonucléotides ont tout d'abord été hybridés de la manière suivante. Un g de chacun de ces deux oligonucléotides ont été mis ensemble dans 40 ml d'un tampon final 50 mM Tris-HCl pH 7.4, 10 mM MgCl₂. Ce mélange a été chauffé à 95°C, puis il a été placé à la température ambiante de manière que la température s'abaisse lentement. Dix ng du mélange d'oligonucléotides hybridés ont été ligaturé avec 200 ng du plasmide pXL2650 linéarisé par BamHI, 30 ml final. Après ligature une aliquote a été transformée dans DH5. Les mélanges de transformation ont été étalés sur milieu L supplémenté avec de l'ampicilline (50 mg/l). Vingt quatre clones ont été digérés par PflMI et BamHI. Un clone a été trouvé qui avait la taille du fragment PflMI-BamHI de 950 pb augmenté de 50 pb. Ce clone a été retenu et nommé pXL2651.

Le plasmide pXL2651 a été purifié selon le kit Wizard Megaprep (Promega Corp., Madison, WI) en suivant les recommandations du fournisseur.

### 6-1.2. Insertion d'une séquence homopurique- homopyrimidique dans le plasmide pXL2649

a) Insertion de nouveaux sites de restriction de part et d'autre de la cassette kanamycine du pXL2649.
   Le plasmide pXL2649, tel qu'il a été décrit exemple 2, a été digéré par EcoRI de manière à ressortir la cassette kanamycine provenant du plasmide pUC4KIXX (PharmaciaBiotech, Uppsala, Suéde). Pour ce faire 5 mg du plasmide pXL2649 ont été digérés par EcoRI. Le fragment de 4,2 kb a été séparé par électrophorèse sur gel d'agarose et purifié par électroélution.
   D'autre part le plasmide pXL1571 a été utilisé. Celui-ci a été construit à partir du plasmide pFR10 (Gene 25 (1983), 71-88), dans lequel le fragment de 1,6 kb provenant du pUC4KIXX, correspondant au gène de la kanamycine, a été inséré en SstI. Ce clonage a permis d'insérer de part et d'autre du gène de la kanamycine 12 nouveaux sites de restriction.
   Cinq micro grammes de pXL1571 ont été dialysés par EcoRI. Le fragment de 1,6 kb correspondant au gène de la kanamycine a été séparé par électrophorèse sur gel d'agarose et, purifié par électroélution. Il a été ensuite ligaturé avec le fragment EcoRI de 4,2 kb du pXL2649. Les clones recombinants ont été sélectionnés après transformation dans E.coli DH5a et sélection pour la résistance à la kanamycine et à l'ampicilline. Le profil de restriction attendu a été observé sur un clone; ce clone plasmidique a été nommé pXL2791.
b) Extraction de la cassette kanamycine du plasmide pXL2791
   Le plasmide pXL2791 a été digéré par SstI de manière à ressortir la cassette kanamycine. Le fragment de 4,2 kb a été séparé par électrophorèse sur gel d'agarose et purifié par le kit de gel extraction Jetsorb (Genomed). Il a ensuite été ligaturé. Les clones recombinants ont été sélectionnés pour la résistance à l'ampicilline après transformation dans E. coli DH5a. Le profil de restriction attendu a été observé sur un clone. Ce clone plasmidique a été nommé pXL2792. Ce clone comprend, entre autre, des sites de restriction SalI et XmaI entre les sites attP et attB.
c) Clonage d'une séquence homopurique-homopyrimidique ainsi que d'une cassette permettant l'expression de la luciférase entre les deux sites attP et attB du plasmide pXL2792
   Le plasmide pXL2727 a été utilisé. Ce plasmide, digéré par XmaI et SalI, permet de ressortir un fragment comprenant : le promoteur pCMV, le gène de la luciférase de photinus pyralis, un site de polyadénylation dérivé de SV40 et une séquence homopurique-homopyrimidique. Cette dernière a été obtenue après hybridation et clonage des deux oligonucléotides suivants :
   6006 : (SEQ ID N.16)
   5'-GATCTGAAGAAGAAGAAGAAGAAGAAGAAGAAGAAGAAGAAGAAGAAGA AGAAGAACTGCAGATCT-3'
   6008 : (SEQ ID N.17)
   5'-GATCAGATCTGCAGTTCTTCTTCTTCTTCTTCTTCT'TCTTCTTCTTCTT CTTCTTCTTCTTCTTCA-3'

La séquence homopurique-homopyrimidique présente sur le pXL2727 a été séquencée par la méthode Sequenase Version 2.0 (United States Biochemical Corporation). Le résultat obtenu montre que la séquence homopurique-homopyrimidique réellement présente sur le plasmide pXL2727 comporte 10 répétitions (GAA-CTT) et non 17 comme le laissait prévoir la séquence des oligonucléotides 6006 et 6008. La séquence réellement présente sur le plasmide pXL2727, lue après séquençage sur le brin correspondant à l'oligonucléotide 6008, est la suivante :
5'-GATCAGATCTGCAGTCTCTTCTTCTTCTTCTTCTTCTTCTTCTTCTTCTCTT CTTCA-3' (SEQ ID N.18)

Un micro gramme de pXL2727 a été digéré par XmaI et SalI. Le fragment de 3,7 kb a été séparé par électrophorèse sur gel d'agarose et purifié par le kit de gel extraction Jetsorb (Genomed). D'autre part 1,7 mg de pXL2792 a été digéré par XmaI et SalI. Le fragment de 4,2 kb a été séparé sur gel d'agarose, purifié par le kit de gel extraction Jetsorb (genomed), et ligaturé avec le fragment XmaI-SalI de 3,7 kb du pXL2727. Les clones recombinants ont été sélectionnés après transformation dans E. coli DH5a et sélection pour la résistance à l'ampicilline. Le profil de restriction attendu a été observé sur un clone; ce clone a été nommé pXL2793. Le plasmide pXL2793 a été purifié en utilisant un gradient de densité en chlorure de césium selon une méthode déja décrite (Maniatis et al., 1989).

### 6-2. Préparation de la colonne permettant de faire des interactions de type triple hélice avec une séquence homopurine-homopyrimidine présente sur le minicercle.

### La colonne a été préparée de la manière suivante :

La colonne utilisée est une colonne HiTrap activée au NHS (N-hydroxysuccinimide, Pharmacia) de 1 ml, connectée sur une pompe péristaltique (débit< 1 ml/min). L'oligonucléotide spécifique utilisé possède un groupement NH₂ en 5'.

Pour le plasmide pXL2651, sa séquence est la suivante :
5'-GAGGCTTCTTCTTCTTCTTCTTCTT-3' (SEQ ID n°5)

Pour le plasmide pXL2793, sa séquence est la suivante (oligo 116418) :
5'-CTTCTTCTTCTTCTTCTTCTT-3' (SEQ ID N.19)

Les tampons utilises sont les suivants :
Tampon de couplage : NaHCO₃ 0,2 M, NaCl 0,5 M, pH 8,3

Tampon de lavage :
Tampon A : éthanolamine 0,5 M, NaCl 0,5 M, pH 8,3
Tampon B : acétate 0,1 M, NaCl 0,5 M pH 4

Tampon de fixation et d'élution :
Tampon F : NaCl 2 M, acétate 0,2 M pH 4,5
Tampon E : Tris 1 M, HCl pH 9, EDTA 0,5 mM

La colonne est préparée de la manière suivante :
La colonne est lavée par 6 ml de HCl 1 mM, puis l'oligonucléotide dilué dans le tampon de couplage (50 nmoles dans 1 ml) est appliquée sur la colonne et laissé 30 minutes à température ambiante. La colonne est lavée par 3 ml de tampon de couplage, puis par 6 ml de tampon A, suivi de 6 ml de tampon B. Ces deux derniers tampons sont appliqués successivement trois fois sur la colonne. l'oligonucléotide est ainsi lié covalemment à la colonne par une liaison CONH. La colonne est stockée à 4°C dans du PBS 0,1 % NaN₃.

### 6-3. Purification d'un minicercle comportant une séquence synthétique homopurique-homopyrimidique, par une interaction de type triple-hélice.

### 6-3.1. Purification du plasmide pXL2651

Le plasmide pXL2651 a été introduit dans la souche D1210HP. Cette souche recombinante [D1210HP (pXL2651)] a été cultivée comme cela est décrit à l'exemple 3, de manière à générer le minicercle contenant le gène de la luciférase de Photinus pyralis. Vingt ml de culture ont été prélevés et centrifugés. Le culot cellulaire est repris dans 1,5 ml de glucose 50 mM, Tris 25 mM HCl pH 8. EDTA 10 mM. La lyse est faite par 2 ml de NaOH 0,2 M, SDS 1 %, et la neutralisation par 1,5 ml d'acétate de potassium 3 M, pH 5. L'ADN est ensuite précipité par 3 ml de propranol-2, le culot est repris dans 0,5 ml d'acétate de sodium 0,2 M pH 5, NaCl 0,1M et chargé sur une colonne d' oligonucléotides capable de former des interactions de type triple hélice avec des séquences poly GAA contenues dans le minicercle, comme décrit précédemment. La colonne ayant été préalablement lavée avec 6 ml de tampon F, la solution contenant le minicercle à purifier après avoir été appliquée sur la colonne est incubée deux heures à température ambiante. La colonne est lavée par 10 ml de tampon F puis l'élution se fait par du tampon E.

On obtient ainsi de l'ADN purifié correspondant au minicercle. Le minicercle obtenu, analysé par électrophorèse sur gel d'agarose et coloration au bromure d'éthidium, se présente sous la forme d'une seule bande d'ADN circulaire super enroulée. Il subsiste dans la préparation moins de 5 % de plasmide pXL2651 de départ.

### 6-3.2. Purification du plasmide pXL2793

Le plasmide pXL2793 de 7,9 kb a été introduit dans la souche D1210HP. Cette souche recombinante a été cultivée comme cela est décrit à l'exemple 3, de manière à générer le minicercle de 4 kb contenant le gène de la luciférase de Photinus pyralis et un plasmide de 3,9 kb. Deux cent ml de culture ont été prélevés et centrifugés. Le culot cellulaire a été traité par le Kit Wizard Megaprep (Promega Corp.,Madison,WI) en suivant les recommandations du fournisseur. L'ADN a été repris dans un volume final de 2 ml de Tris 1mM. EDTA 1 mM, pH 8. Deux cent cinquante micro litres de cet échantillon plasmidique ont été dilués avec du tampon F dans un volume final de 2,5 ml. La colonne a été préalablement lavée avec 6 ml de tampon F. La totalité de l'échantillon dilué a été chargé sur une colonne d'oligonucléotide capable de former des interactions de type triple hélice avec des séquences poly GAA contenues dans le minioercle, préparée comme décrit précédemment. Après lavage par 10 ml de tampon F, l'élution se fait par du tampon E. L'échantillon élué est récupéré par fraction de 1 ml.

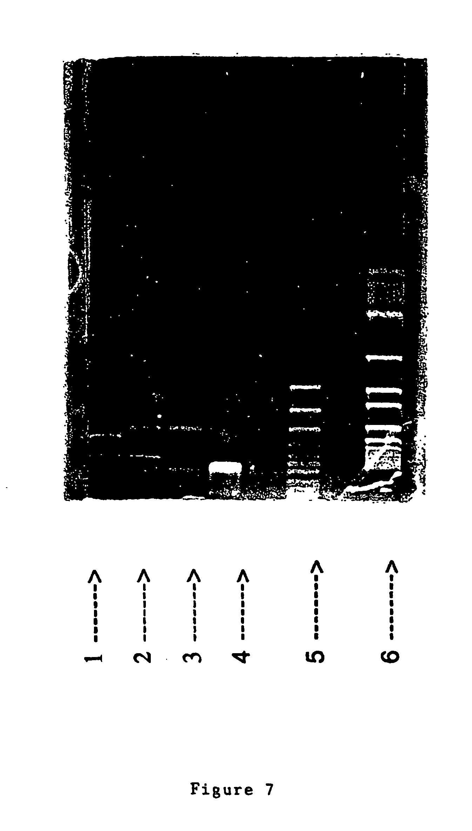

Par cette méthode on obtient de l'ADN purifié correspondant au minicercle généré à partir de pXL2793. L'échantillon d'ADN élué de la colonne a été analysé par électrophorèse sur gel d'agarose et coloration au bromure d'éthidium et par restriction enzymatique. Pour ce faire les fractions éluées se révélant contenir de l'ADN par dosage à DO₂₆₀ nm ont été dialysées 24 heures contre du Tris 1mM, EDTA 1mM, puis précipitées à l'isopropanol et repris dans 200 ml d'H₂O. Quinze micro litres de l'échantillon ainsi obtenu ont été digérés par SalI, ce site de restriction étant présent sur le minicercle et pas sur le plasmide de 3,9 kb généré par la recombinaison, ou par XmnI, ce site de restriction étant présent sur le plasmide de 3,9 kb généré par la recombinaison et pas sur le minicercle. Le résultat obtenu est présenté sur la figure 7. montrant que le minicercle a été purifié du plasmide recombinant.

### Exemple 7 : Transfection in vivo de cellules de mammifères par un minicercle.

Cet exemple décrit le transfert d'un minicercle codant pour le gène de la luciférase dans le cerveau de souris nouveau-nées. Le minicercle (30 µg) est dilué dans du NaCl 150 mM stérile, à une concentration de 1 µg/µl. On rajoute ensuite un transfectant synthétique, tel que le dioctadécylamidoglycylspermine (DOGS), dans un rapport de charges positives/négatives inférieur ou égal à 2. Le mélange est vortexé et 2 µg d'ADN sont injectés dans le cortex cérébral de souris nouveau-nées anesthésiées, à l'aide d'un micromanipulateur et d'une microseringue. Les cerveaux sont prélevés 48 heures plus tard, homogénéisés, centrifugés et le surnageant est utilisé pour le dosage de la luciférase par les protocoles décrits (tels le kit Promega).

### Exemple 8 : Utilisation du locus par de RK2 pour diminuer la présence de topo-isomères de minicercle ou de miniplasmide

Cet exemple met en évidence la présence de formes topologiques dérivées i) du plasmide possédant les séquences attP et attB en orientation directe, ii) du minicercle ou iii) du miniplasmide, après action de l'intégrase du bactériophage 1 chez E. coli. Cet exemple montre aussi que ces formes topologiques ou oligomériques peuvent être resolues par l'utilisation du locus par de RK2 (Gerlitz et coll. 1990 J. Bacteriol. 172p6194). En effet, ce locus contient en particulier le gène parA codant pour une résolvase agissant au site mrs (système de résolution de multimères) (EberI et coll. 1994 Mol. Microbio1.12 p.131).

### 8.1.-Construction des plasmides pXL2777 et pXL2960

Les plasmides pXL2777 et pXL2960 sont dérivés du vecteur pXL2776 et possèdent en commun le réplicon minimal de ColE1, le gène du transposon Tn5 codant pour la résistance à la kanamycine et les séquences attP et attB du bactériophage 1 en orientation directe. Ces plasmides sont différents au niveau des gènes insérés entre les séquences attP et attB en particulier le pXL2777 contient la cassette omegon (codant pour le gène de résistance à la spectinomycine) alors que le plasmide pXL2960 porte le locus par de RK2.

### 8-1.1 Vecteur minimal pXL2658

Le vecteur pXL2658 (2,513 kb) possède le réplicon minimal de ColE1 issu de pBluescript (ori) et le gène du transposon Tn5 codant pour la résistance à la kanamycine (Km) pour marqueur de sélection. Après avoir rendu l'extrémité BsaI franche par action de Ia Klenow, le fragment BgauI-PvuII de 1,15 kb de pBKS+ (provenant de Stratagene) a été cloné avec le fragment SmaI de 1,2 kb du pUC4KIXX (provenant de Pharmacia) pour générer le plasmide pXL2647. Les oligonucléotides 5542 5'(AGC TTC TCG AGC TGC AGG ATA TCG AAT TCG GAT CCT CTA GAG CGG CCG CGA GCT CC)3' (SEQ ID N.20) et 5543 5'(AGC TGG AGC TCG CGG CCG CTC TAG AGG ATC CGA ATT CGA TAT CCT GCA GCT CGA GA)3' (SEQ ID N.21) ont été hybridés entre eux puis clonés au site HindIII du pXL2647 ainsi est construit le pXL2658. Sur ce plasmide, le multisite est SstI, NotI, XbaI, BamHI, EcoRI, EcoRV, PstI, XhoI, et HindIII entre l'origine de réplication et le gène codant pour la résistance à la kanamycine.

### 8-1.2. Vecteur pXL2776 contenant les séquences attP et attB du phage 1

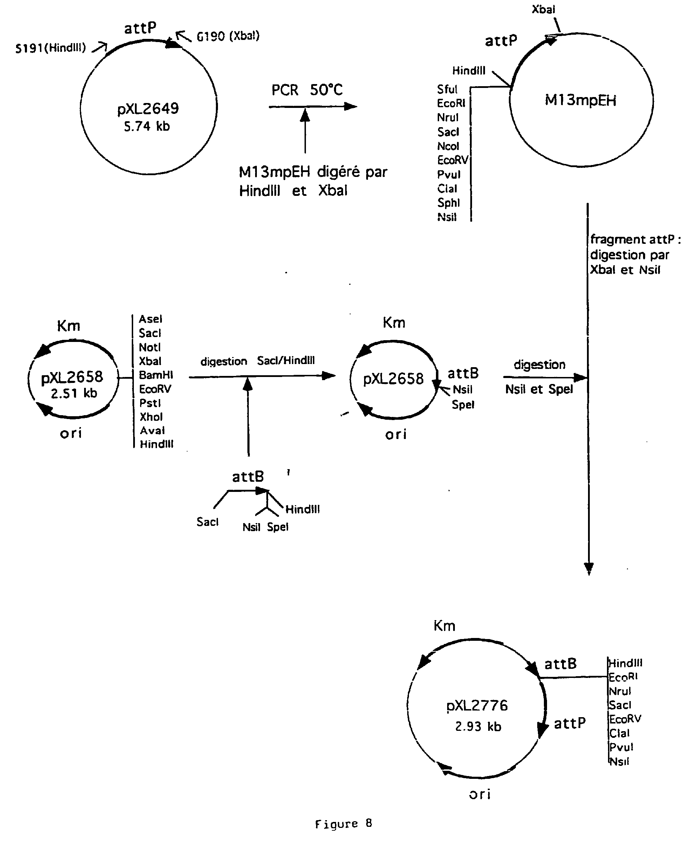

Le vecteur pXL2776 (2,93 kb) possède le réplicon minimal de ColE1 issu de pBluescript, le gène codant pour la résistance à la kanamycine et les séquences attP et attB du bactériophage 1 en orientation directe, voir figure 8. La séquence attB de 29 pb (Mizuuchi et coll. 1980 Proc. Natl. Acad. Sci. USA 77 p3220) a été introduite entre les sites de restriction SacI et HindIII du pXL2658 après avoir hybridé l'oligonucléotide sens 6194 5'(ACT AGT GGC CAT GCA TCC GCT CAA GTT AGT ATA AAA AAG CAG GCT TCA G)3' (SEQ ID N.22) avec l'oligonucléotide antisens 6195 5' (AGC TCT GAA GCC TGC TTT TTT ATA CTA ACT TGA GCG GAT GCA TGG CCA CTA GTA GCT)3' (SEQ ID N.23) de telle sorte que les sites SacI et HindIII ne soient plus reconstitués après clonage. Ce plasmide, dont la séquence a été vérifiée en attB, est alors digéré par SpeI et NsiI pour y introduire la séquence attP encadrée par les sites de restriction NsiI et XbaI et générer alors le plasmide pXL2776. La séquence attP a été obtenue par amplification PCR en utilisant le plasmide pXL2649 (décrit dans l'exemple 2) comme matrice, les oligonucléotides 6190 sens 5'(GCG TCT AGA ACA GTA TCG TGA TGA CAG AG)3' (SEQ ID N.24) et 6191 antisens 5'(GCC AAG CTT AGC TTT GCA CTG GAT TGC GA)3' (SEQ ID N.25) et en effectuant 30 cycles au cours desquels la température d'hybridation est de 50°C . Le produit PCR digéré par les sites XbaI et HindIII a été cloné dans le phage M13mpEH entre les sites XbaI et HindIII. La séquence amplifiée est identique à la séquence de attP décrite dans Lambda II (édité par R.W. Hendrix, J. W. Roberts, F.W. Stahl, R.A. Weisberg; Cold Spring Harbor Laboratory 1983) entre les positions 27480 et 27863.

### 8-1.3. Plasmide pXL2777

Le plasmide pXL2777 (6,9 kb) possède le réplicon minimal de ColE1 issu de pBluescript, le gène codant pour la résistance à la kanamycine, les séquences attP et attB du bactériophage 1 en orientation directe et séparées par le gène sacB codant pour la levanesucrase de B. subtilis (P. Gay et coll. 1983 J. Bacteriol. 153 p1424) et l'omegon Sp codant pour le gène de résistance à la spectinomycine Sp et la streptomycine Sm (P. Prentki et coll. 1984 Gene 29 p303). La cassette sacB-Sp ayant des extrémités de clonage EcoRV et NsiI provient du plasmide pXL2757 (FR95/01632) et a été clonée entre les sites EcoRV et NsiI du pXL2776 pour former le pXL2777.

### 8-1.4. Plasmide pXL2960

Le plasmide pXL2960 (7,3 kb) possède le réplicon minimal de ColE1 issu de pBluescript, le gène codant pour la résistance à la kanamycine, les séquences attP et attB du bactériophage 1 en orientation directe et séparées par i) le gène sacB codant pour la levanesucrase de B. subtilis (P. Gay et coll. 1983 J. Bacteriol. 153 p1424) et ii) le locus par de RK2 (Gerlitz et coll. 1990 J. Bacteriol. 172p6194). La cassette par ayant des extrémités BamHI provient du plasmide pXL2433 (PCT/FR95/01178) et a été introduite entre les sites BamHI du pXL2777 pour générer le pXL2960.

### 8-2. Resolution de topo-isomères de minicercle ou de miniplasmide

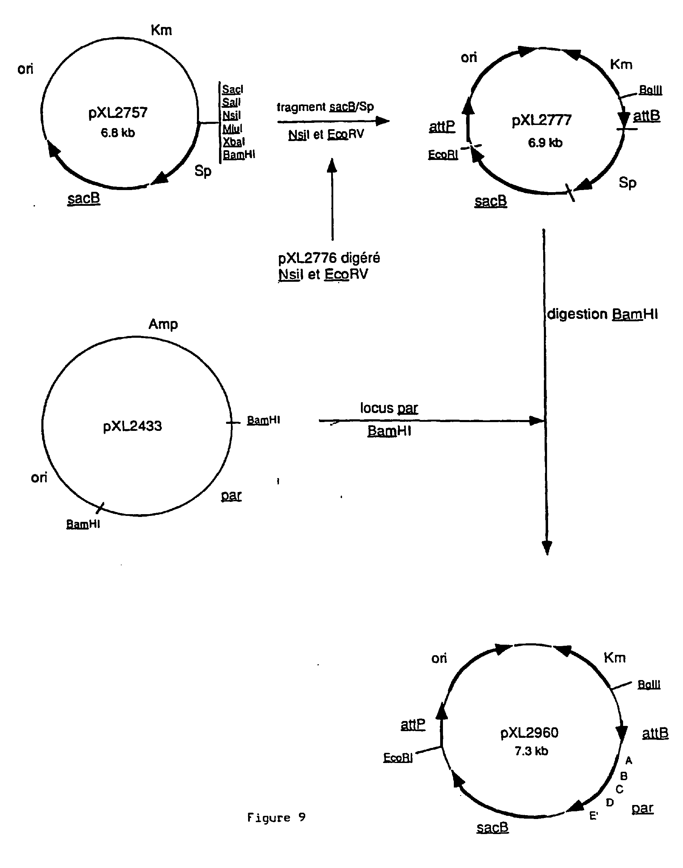

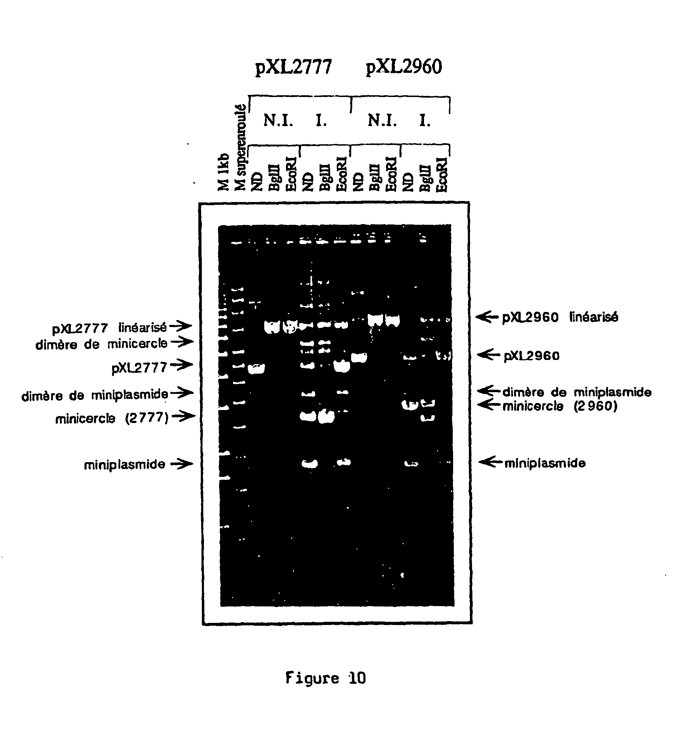

Les plasmides pXL2777 et pXL2960 ont été introduits par transformation dans la souche de E. coli D1210HP. Les transformants ont été sélectionnés et analysés comme cela a été décrit dans l'exemple 2 avec les modifications suivantes : l'expression de l'intégrase a été induite à 42°C pendant 15 min lorsque la densité optique à 610 nm des cellules est de 1,8 puis les cellules sont incubées à 30°C pendant 30 min, voir figure 9 ou pendant une durée variant de 2 minutes à 14 heures (O/N), voir figure 10. L'ADN plasmidique provenant des cultures non induite et induite a été analysé sur gel d'agarose avant ou après digestion par un enzyme de restriction unique à la partie minicercle (EcoRI) ou miniplasmide (BglII), voir figure Y, ou après action de l'ADN topo-isomèrase A ou la gyrase de E. coli. Les formes dimères surenroulées de minicercle ou miniplasmide sont clairement mises en évidence par i) leur poids moléculaire, ii) leur linéarisation par l'enzyme de restriction, iii) leur changement de topologie par l'action de la topo-isomèrase A (dimère relaché) ou de la gyrase (dimère supersuperenroulé), iv) l'hybridation spécifique avec un fragment interne propre au minicercle ou au miniplasmide. D'autres formes topologiques de poids moléculaires plus élevées que celui du plasmide initial sont issues du plasmide initial ou du minicercle ou du miniplasmide puisqu'elles disparaissent après digestion par l'enzyme de restriction unique à la partie minicercle (EcoRI) ou miniplasmide (BglII). Ces formes sont beaucoup moins abondantes avec le pXL2960 qu'avec le pXL2777 comme plasmide initial, voir figure 10. En particulier la forme dimère de minicercle est présente de manière non négligeable avec le plasmide pXL2777 alors qu'elle est invisible avec le plasmide pXL2960, lorsque les cellules sont incubées au moins 30 min à 30°C, voir figures 9 et 10. Il est à noter que des dimères de minicercle sont observés en début de cinétique avec le pXL2960 (2 à 10 min.), puis sont ensuite résolus (après 30 min.), voir figure 10. Par conséquent le locus par conduit à une diminution significative des formes oligomériques/topologiques résultant de l'action de l'intégrase du bactériophage 1 chez E. coli sur les plasmides contenant les séquences attP et attB en orientation directe.

### IDENTIFICATION DES SEQUENCES NUCLEOTIDIOUES

SEQ ID n° 1 : Oligonucléotide 5476 :
   5'-AATTGTGAAGCCTGCTTTTTTATACTAACTTGAGCGG-3'
SEQ ID n° 2 : Oligonucléotide 5477
   5'-AATTCCGCTCAAGTTAGTATAAAAAAGCAGGCTTCAC-3'
SEQ ID n° 3 : 1 Oligonucléotide 4957 :
   5'-GATCCGAAGAAGAAGAAGAAGAAGAAGAAGAAGAAGAAGAAGAAGAAGAAGAAGAAC-3'
SEQ ID n° 4 : Oligonucléotide 4958 :
   5'-GATCGTTCTTCTTCTTCTTCTTCTTCTTCTTCTTCTTCTTCTTCTTCTTCTTCTTCG-3'
SEQ ID n° 5 :oligonucléotide poly-CTT :
   5'-GAGGCTTCTTCTTCTTCTTCTTCTT-3'
SEQ ID n° 6 : (Séquence attB du phage lambda) :
   5'-CTGCTTTTTTATACTAACTTG-3'
SEQ ID n° 7 : (Séquence attP du phage lambda) :
   5'-CAGCTTTTTTATACTAAGTTG-3'
SEQ ID n° 8 : (Séquence attB du phage P22) :
   5'-CAGCGCATTCGTAATGCGAAG-3'
SEQ ID n° 9 : (Séquence attP du phage P22) :
   5'-CTTATAATTCGTAATGCGAAG-3'
SEQ ID n° 10 : (Séquence attB du phage F80) :
   5'-AACACTTTCTTAAATGGTT-3'
SEQ ID n° 11 :(Séquence attP du phage F80) :
   5'-AACACTTTCTTAAATTGTC-3'
SEQ ID n° 12 (Séquence attB du phage HP1) :
   5'-AAGGGATTTAAAATCCCTC-3'
SEQ ID n° 13 :(Séquence attP du phage HP1) :
   5'-ATGGTATTTAAAATCCCTC-3'
SEQ ID n° 14 : (Séquence att du plasmide pSAM2) :
   5'-TTCTCTGTCGGGGTGGCGGGATTTGAACCCACGACCTCTTCGTCCCGAA-3'
SEQ ID n° 15 :(Séquence de reconnaissance de la résolvase du transposon Tn3) :
   5'-CGTCGAAATATTATAAATTATCAGACA-3'
SEQ ID n° 16 : oligonucléotide 6006 :
   5'-GATCTGAAGAAGAAGAAGAAGAAGAAGAAGAAGAAGAAGAAGAAGAAGAAGAAG AACTGCAGATCT-3'
SEQ ID n° 17 : oligonucléotide 6008 :
   5'-GATCAGATCTGCAGTTCTTCTTCTTCTTCTTCTTCTTCTTCTTCTTCTTCTTCT TCTTCTTCTTCA-3'
SEQ ID n° 18 :(Séquence présente sur le plasmide pXL2727 correspondant à l'oligonucléotide 6008) :
   5'-GATCAGATCTGCAGTCTCTTCTTCTTCTTCTTCTTCTTCTTCTTCTTCTCTTCT TCA-3'
SEQ ID n° 19 : (oligonucléotide 116418) :
   5'-CTTCTTCTTCTTCTTCTTCTT-3'
SEQ ID n°20 :(oligonucléotide 5542) :
   5'-AGCTTCTCGAGCTGCAGGATATCGAATTCGGATCCTCTAGAGCGGCCGCGAGCT CC-3'
SEQ ID n° 21 :(oligonucléotide 5543) :
   5'-AGCTGGAGCTCGCGGCCGCTCTAGAGGATCCGAATTCGATATCCTGCAGCTCGA GA-3'
SEQ ID n° 22 : oligonucléotide sens 6194 :
   5'-ACTAGTGGCCATGCATCCGCTCAAGTTAGTATAAAAAAGCAGGCTTCAG-3'
SEQ ID n° 23 : oligonucléotide antisens 6195 :
   5'-AGCTCTGAAGCCTGCTTTTTTATACTAACTTGAGCGGATGCATGGCCACTAGTA GCT-3'
SEQ ID n° 24 : oligonucléotide sens 6190 :
   5'-GCGTCTAGAACAGTATCGTGATGACAGAG-3'
SEQ ID n° 25 : oligonucléotide antisens 6191 :
   5'-GCCAAGCTTAGCTTTGCACTGGATTGCGA-3'

### Références bibliographiques

Ausubel et al. Current protocols in molecular biology 1987-1988. John Willey and Sons, New York.
Behr J.P. 1993. Acc. Chem. Res. 26:274-278.
Casadaban et al. 1983. Methods Enzymol. 100, 293-308.
Cotten et al. E. 1993. Curr. Biol. 4:705-710.
Giles, J. W. 1985. Am. Biotechnol., Nov./Dec.
Hasan et al. 1987. Gene 56:145-151.
Jain, R. K. 1987. Cancer Res. 47:3039-3051.
Landford et al 1986. Cell 46:575-582.
Landy, A. 1989. Ann. Rev. Biochem. 58:913-949.
Maniatis, T., E. F . Fritsch, and J. Sambrook. 1989. Molecular cloning : a laboratory manual, second edition. Cold Spring Harbor Laboratory, Cold Spring Harbor Laboratory Press, New York.
Nabel et al. 1992. Human Gene Therapy 3:399-410.
Podhajska et al. 1985. Gene 40:163:168.
Sadler et al. 1980. Gene, 8:279-300.
Sinha et al. 1984. Acids Res., 12, 4539-4557.
Stark et al. 1992. Trends Genet. 8:432-439.
Viera et al. 1982. Gene, 19, 259-268.
Wils et al. Biochem. Pharmacol. 48:1528-1530.

## Revendications

1. Molécule d'ADN double brin **caractérisée en ce que**:
- elle est sous forme circulaire et super enroulée,
- elle comprend une cassette d'expression constituée d'un gène d'intérêt thérapeutique, vaccinal, agronomique ou vétérinaire, sous contrôler d'un promoteur et d'un terminateur transcriptionnels actifs dans les cellules mammifères,
- elle est dépourvue d'origine de réplication
- elle est dépourvue de gène marqueur, et,
- elle comprend une région résultant de la recombinaison site spécifique entre deux séquences, ladite région étant située hors de la cassette d'expression.

2. Molécule selon la revendication 1 **caractérisée en ce qu'**elle contient en outre une séquence capable de former une triple hélice par hybridation avec un oligonucléotide spécifique.

3. Molécule selon la revendication 2 **caractérisée en ce que** la séquence capable de former une triple hélice comprend de 5 à 30 paires de bases.

4. Molécule selon la revendication 2 ou 3 **caractérisée en ce que** la séquence capable de former une triple hélice est une séquence homopurique homopyrimidique.

5. Molécule selon la revendication 1 **caractérisée en ce qu'**elle comprend une séquence résultant de la recombinaison site spécifique entre deux séquences d'attachement att ou entre deux séquences de reconnaissance de la résolvase d'un transposon ou parA de RK2.

6. Molécule selon l'une des revendications précédentes **caractérisée en ce qu'**elle comprend en outre une séquence mrs issue du locus par de RK2, permettant de résoudre les multimères.

7. Molécule selon l'une des revendications précédentes **caractérisée en ce que** le gène d'intérêt est un acide nucléique codant pour un produit thérapeutique, vaccinal, agronomique ou vétérinaire.

8. Molécule selon l'une des revendications précédentes **caractérisée en ce qu'**elle est obtenue par excision à partir d'un plasmide ou d'un chromosome, par recombinaison site spécifique.

9. ADN recombinant comprenant une partie non thérapeutique et une partie thérapeutique ou cassette d'expression constituée d'un gène d'intérêt thérapeutique, vaccinal, agronomique ou vétérinaire, sous contrôle d'un promoteur et d'un terminateur transcriptionnels actifs dans les cellules mammifères encadrée par deux séquences permettant une recombinaison site spécifique, positionnées en orientation directe, la recombinaison par excision des parties non thérapeutiques permettant de générer la molécule selon l'une des revendications 1 à 8.

10. ADN recombinant selon la revendication 9 **caractérisé en ce qu'**il s'agit d'un plasmide réplicatif comprenant
a) une origine de réplication et éventuellement un gène marqueur dans la partie non thérapeutique,
b) deux séquences permettant une recombinaison site spécifique positionnées en orientation directe, et,
c) placés entre lesdites séquences b), une partie thérapeutique ou cassette d'expression constituée d'un gène d'intérêt thérapeutique, vaccinal, agronomique ou vétérinaire, sous contrôle d'un promoteur et d'un terminateur transcriptionnels actifs dans les cellules mammifères.

11. ADN recombinant selon les revendications 9 et 10 **caractérisé en ce que** les séquences permettant la recombinaison site spécifique sont des séquences capables de recombiner spécifiquement en présence d'une recombinase.

12. ADN recombinant selon la revendication 11 **caractérisé en ce que** la recombinase est choisie parmi les recombinases de la famille de l'intégrase du phage lambda et de la famille de la résolvase du transposon Tn3.

13. ADN recombinant selon l'une des revendications 9 à 12 **caractérisé en ce que** les séquences permettant la recombinaison site spécifique sont dérivées d'un bactériophage.

14. ADN recombinant selon la revendication 13 **caractérisé en ce que** les séquences permettant la recombinaison site spécifique sont constituées par les séquences d'attachement att d'un bactériophage.

15. ADN recombinant selon la revendication 14 **caractérisé en ce que** les séquences permettant la recombinaison site spécifique sont constituées par les séquences d'attachement du bactériophage lambda, P22, Φ80, P1, HP1 ou encore du plasmide pSAM2 ou 2µ.

16. ADN recombinant selon la revendication 15 **caractérisé en ce que** les séquences permettant la recombinaison site spécifique comprennent tout ou partie des séquences SEQ ID n° 1, 2, 6, 7, 8, 9, 10, 11, 12, 13 ou 14.

17. Plasmide **caractérisé en ce qu'**il comprend
(a) une origine de réplication bactérienne et éventuellement un gène marqueur dans la partie non thérapeutique ;
(b) les séquences attP et attB d'un bactériophage sélectionné parmi les phages lambda, P22, Φ80, P1, HP1 ou du plasmide pSAM2 ou 2 µ, positionnées en orientation directe; et
(c) placés entre lesdites séquences, une partie thérapeutique ou cassette d'expression constituée d'un gène d'intérêt thérapeutique, vaccinal, agronomique ou vétérinaire sous contrôle d'un promoteur et d'un terminateur transcriptionnels actifs dans les cellules mammifères.

18. Plasmide selon la revendication 17 **caractérisé en ce que** les séquences permettant la recombinaison site spécifique sont constituées par les séquences d'attachement du bactériophage lambda.

19. ADN recombinant selon la revendication 13 **caractérisé en ce que** les séquences permettant la recombinaison site spécifique sont dérivées du bactériophage P1.

20. Plasmide **caractérisé en ce qu'**il comprend :
(a) une origine de réplication bactérienne et éventuellement un gène marqueur dans la partie non thérapeutique;
(b) les séquences répétées inversées du bactériophage P1 (région loxP) positionnées en orientation directe ; et,
(c) placés entre lesdites séquences (b), une partie thérapeutique ou cassette d'expression constituée d'un gène d'intérêt thérapeutique, vaccinal, agronomique ou vétérinaire sous contrôle d'un promoteur et d'un terminateur transcriptionnels actifs dans les cellules mammifères.

21. ADN recombinant selon l'une des revendications 9 à 12 **caractérisé en ce que** les séquences permettant la recombinaison site spécifique sont dérivées d'un transposon.

22. ADN recombinant selon la revendication 21 **caractérisé en ce que** les séquences permettant la recombinaison site spécifique sont constituées par des séquences de reconnaissance de la résolvase du transposon Tn3, Tn21 ou Tn522.

23. ADN recombinant selon la revendication 22 **caractérisé en ce que** les séquences permettant la recombinaison site spécifique comprennent tout ou partie de la séquence SEQ 1D N° 15.

24. ADN recombinant selon l'une des revendications 9 à 12 **caractérisé en ce que** les séquences permettant la recombinaison site spécifique sont dérivées de la région par du plasmide RP4.

25. ADN recombinant selon l'une des revendications 9 à 24 **caractérisé en ce qu'**il comprend en outre une séquence capable de former une triple hélice par hybridation avec un oligonucléotide spécifique.

26. Plasmide **caractérisé en ce qu'**il comprend :
a) une origine de réplication et éventuellement un gène marqueur dans la partie non thérapeutique,
b) deux séquences permettant une recombinaison site spécifique positionnées en orientation directe, et,
c) placés entre lesdites séquences b), une partie thérapeutique ou cassette d'expression constituée d'un ou plusieurs gènes d'intérêt thérapeutique, vaccinal, agronomique ou vétérinaire et d'une séquence capable de former une triple hélice par hybridation avec un oligonucléotide spécifique.

27. Plasmide **caractérisé en ce qu'**il comprend :
a) une origine de réplication et éventuellement un gène marqueur dans la partie non thérapeutique,
b) deux séquences permettant une recombinaison site spécifique positionnées en orientation directe, et,
c) placés entre lesdites séquences b), une partie thérapeutique ou cassette d'expression constituée d'un ou plusieurs gènes d'intérêt thérapeutique, vaccinal, agronomique ou vétérinaire et d'une séquence mrs issue du locus par de RK2, permettant de résoudre les multiméres.

28. Plasmide **caractérisé en ce qu'**il comprend :
a) une origine de réplication et éventuellement un gène marqueur dans la partie non thérapeutique,
b) deux séquences permettant une recombinaison site spécifique positionnées en orientation directe, et,
c) placés entre lesdites séquences b), une partie thérapeutique ou cassette d'expression constituée d'un ou plusieurs gènes d'intérêt thérapeutique, vaccinal, agronomique ou vétérinaire, d'une séquence mrs issue du locus par de RK2, permettant de résoudre les multiméres et une séquence capable de former une triple hélice par hybridation avec un oligonucléotide spécifique.

29. Plasmide **caractérisé en ce qu'**il comprend :
a) une origine de réplication et éventuellement un gène marqueur dans la partie non thérapeutique,
b) deux séquences permettant une recombinaison site spécifique intégrase dépendante positionnées en orientation directe et deux séquences permettant une recombinaison site spécifique résolvase dépendante positionnées à côté des deux premières et également en orientation directe, et,
c) placés entre lesdites séquences b), une partie thérapeutique ou cassette d'expression constituée d'un ou plusieurs gènes d'intérêt thérapeutique, vaccinal, agronomique ou vétérinaire et éventuellement d'une séquence capable de former une triple hélice par hybridation avec un oligonucléotide spécifique.

30. Plasmide selon les revendications 26, 28 et 29 **caractérisé en ce que** la séquence capable de former une triple hélice est définie comme dans les revendications 3 et 4.

31. Cellule recombinante contenant un plasmide ou un ADN recombinant selon la revendication 13, 26, 28 ou 29.

32. Cellule recombinante comprenant, inséré dans son génome, une ou plusieurs copies d'un ADN recombinant selon la revendication 9.

33. Cellule recombinante selon la revendication 31 ou 32 **caractérisée en ce qu'**il s'agit d'une bactérie.

34. Cellule recombinante selon la revendication 31 ou 32 **caractérisée en ce qu'**il s'agit, d'une cellule eucaryote.

35. Cellule recombinante selon la revendication 33 **caractérisée en ce qu'**il s'agit de la bactérie *E. coli* D1 21 0HP.

36. Composition pharmaceutique comprenant une molécule d'ADN au moins selon l'une des revendications 1 à 8.

37. Procédé de préparation d'une molécule d'ADN selon l'une des revendications 1 à 8 **caractérisé en ce qu'**une culture de cellules hôtes contenant un ADN recombinant tel que défini dans la revendication 9 est mise en contact avec la recombinase permettant d'induire la recombinaison site spécifique *in vivo.*

38. Procédé selon la revendication 37 **caractérisé en ce que** la culture de cellules hôtes est une culture de cellules selon la revendication 31.

39. Procédé selon la revendication 37 **caractérisé en ce que** la culture de cellules hôtes est une culture de cellules selon la revendication 32.

40. Procédé selon l'une des revendications 37 à 39 **caractérisé en ce que** la mise en contact avec la recombinase est effectuée par transfection ou infection de la culture de cellules avec un plasmide ou un phage contenant le gène de la dite recombinase.

41. Procédé selon l'une des revendications 37 à 39 **caractérisé en ce que** la mise en contact avec la recombinase est effectuée par induction de l'expression d'un gène codant pour la dite recombinase, présent dans la cellule hôte.

42. Procédé selon la revendication 41 **caractérisé en ce que** la cellule hôte contient, intégrée dans son génome, le gène de la recombinase ayant une expression thermorégulée et la mise en contact avec la recombinase est effectuée par culture à la température d'induction.

43. Procédé selon la revendication 42 **caractérisé en ce que** la cellule hôte utilisée contient un phage lysogène intégré dans son génome, contenant le gène de ladite recombinase.

44. Procédé de préparation d'une molécule d'ADN selon l'une des revendications 1 à 8 **caractérisé en ce qu'**une préparation d'ADN recombinant selon la revendication 11 est mise en contact avec la recombinase permettant d'induire la recombinaison site spécifique in vitro.

45. Procédé selon la revendication 37 ou 44 **caractérisé en ce qu'**il comprend une étape supplémentaire de purification de la molécule selon l'une des revendications 1 à 8.

46. Procédé selon la revendication 45 **caractérisé en ce que** la purification comprend une étape de mise en contact d'une solution contenant la molécule selon l'une des revendications 1 à 8 avec un ligand spécifique, éventuellement greffé sur un support.

47. Procédé selon la revendication 46 **caractérisé en ce que** la solution contenant la molécule selon l'une des revendications 1 à 8 est mise en contact avec un oligonucléotide, éventuellement greffé sur un support, capable de former par hybridation une triple hélice avec une séquence spécifique présente dans ladite molécule selon l'une des revendications 1 à 8.

## Claims

1. Double-stranded DNA molecule, **characterized in that**:
- it is in circular and supercoiled form,
- it comprises an expression cassette consisting of a gene of therapeutic, vaccinal, agricultural or veterinary interest under the control of a transcription promoter and a transcription terminator which are active in mammalian cells,
- it lacks an origin of replication,
- it lacks a marker gene, and
- it comprises a region resulting from the site-specific recombination between two sequences, said region being located outside the expression cassette.

2. Molecule according to claim 1, **characterized in that** it contains, in addition, a sequence capable of forming a triple helix by hybridization with a specific oligonucleotide.

3. Molecule according to claim 2, **characterized in that** the sequence capable of forming a triple helix comprises from 5 to 30 base pairs.

4. Molecule according to claim 2 or 3, **characterized in that** the sequence capable of forming a triple helix is a homopurine-homopyrimidine sequence.

5. Molecule according to claim 1, **characterized in that** it comprises a sequence resulting from the site-specific recombination between two att attachment sequences or between two recognition sequences of the resolvase of a transposon or parA of RK2.

6. Molecule according to one of the preceding claims, **characterized in that** it comprises, in addition, an mrs sequence originating from the par locus of RK2, permitting the resolution of multimers.

7. Molecule according to one of the preceding claims, **characterized in that** the gene of interest is a nucleic acid coding for a therapeutic, vaccinal, agricultural or veterinary product.

8. Molecule according to one of the preceding claims, **characterized in that** it is obtained by excision from a plasmid or chromosome, by site-specific recombination.

9. Recombinant DNA, comprising a non-therapeutic part and a therapeutic part or expression cassette consisting of a gene of therapeutic, vaccinal, agricultural or veterinary interest under the control of a transcription promoter and a transcription terminator which are active in mammalian cells, flanked by two sequences permitting a site-specific recombination, positioned in the direct orientation, the recombination by excision of the non-therapeutic parts making it possible to generate the molecule according to one of claims 1 to 8.

10. Recombinant DNA according to claim 9, **characterized in that** it is a replicative plasmid comprising:
a) an origin of replication and optionally a marker gene in the non-therapeutic part,
b) two sequences permitting a site-specific recombination, positioned in the direct orientation, and,
c) placed between said sequences b), a therapeutic part or expression cassette consisting of a gene of therapeutic, vaccinal, agricultural or veterinary interest under the control of a transcription promoter and a transcription terminator which are active in mammalian cells.

11. Recombinant DNA according to claims 9 and 10, **characterized in that** the sequences permitting site-specific recombination are sequences capable of recombining specifically in the presence of a recombinase.

12. Recombinant DNA according to claim 11, **characterized in that** the recombinase is chosen from the recombinases of the integrase family of phage lambda and of the resolvase family of the transposon Tn3.

13. Recombinant DNA according to one of claims 9 to 12, **characterized in that** the sequences permitting site-specific recombination are derived from a bacteriophage.

14. Recombinant DNA according to claim 13, **characterized in that** the sequences permitting site-specific recombination consist of the att attachment sequences of a bacteriophage.

15. Recombinant DNA according to claim 14, **characterized in that** the sequences permitting site-specific recombination consist of the attachment sequences of bacteriophage lambda, P22, Φ80, P1 or HP1 or alternatively of plasmid pSAM2 or the 2µ plasmid.

16. Recombinant DNA according to claim 15, **characterized in that** the sequences permitting site-specific recombination comprise all or part of the sequences SEQ ID No.1, 2, 6, 7, 8, 9, 10, 11, 12, 13 or 14.

17. Plasmid, **characterized in that** it comprises:
(a) a bacterial origin of replication and optionally a marker gene in the non-therapeutic part;
(b) the attP and attB sequences of a bacteriophage selected from the phages lambda, P22, Φ80, P1 and HP1 or of plasmid pSAM2 or the 2µ plasmid, positioned in the direct orientation; and,
(c) placed between said sequences, a therapeutic part or expression cassette consisting of a gene of therapeutic, vaccinal, agricultural or veterinary interest under the control of a transcription promoter and a transcription terminator which are active in mammalian cells.

18. Plasmid according to claim 17, **characterized in that** the sequences permitting site-specific recombination consist of the attachment sequences of bacteriophage lambda.

19. Recombinant DNA according to claim 13, **characterized in that** the sequences permitting site-specific recombination are derived from bacteriophage P1.

20. Plasmid, **characterized in that** it comprises:
(a) a bacterial origin of replication and optionally a marker gene in the non-therapeutic part;
(b) the inverted repeat sequences of bacteriophage P1 (loxP region), positioned in the direct orientation; and,
(c) placed between said sequences (b), a therapeutic part or expression cassette consisting of a gene of therapeutic, vaccinal, agricultural or veterinary interest under the control of a transcription promoter and a transcription terminator which are active in mammalian cells.

21. Recombinant DNA according to one of claims 9 to 12, **characterized in that** the sequences permitting site-specific recombination are derived from a transposon.

22. Recombinant DNA according to claim 21, **characterized in that** the sequences permitting site-specific recombination consist of the recognition sequences of the resolvase of the transposon Tn3, Tn21 or Tn522.

23. Recombinant DNA according to claim 22, **characterized in that** the sequences permitting site-specific recombination comprise all or part of the sequence SEQ ID No.15.

24. Recombinant DNA according to one of claims 9 to 12, **characterized in that** the sequences permitting site-specific recombination are derived from the par region of plasmid RP4.

25. Recombinant DNA according to one of claims 9 to 24, **characterized in that** it comprises, in addition, a sequence capable of forming a triple helix by hybridization with a specific oligonucleotide.

26. Plasmid, **characterized in that** it comprises:
a) an origin of replication and optionally a marker gene in the non-therapeutic part,
b) two sequences permitting a site-specific recombination, positioned in the direct orientation, and,
c) placed between said sequences b), a therapeutic part or expression cassette consisting of one or more genes of therapeutic, vaccinal, agricultural or veterinary interest and of a sequence capable of forming a triple helix by hybridization with a specific oligonucleotide.

27. Plasmid, **characterized in that** it comprises:
a) an origin of replication and optionally a marker gene in the non-therapeutic part,
b) two sequences permitting a site-specific recombination, positioned in the direct orientation, and,
c) placed between said sequences b), a therapeutic part or expression cassette consisting of one or more genes of therapeutic, vaccinal, agricultural or veterinary interest and of an mrs sequence originating from the par locus of RK2, permitting the resolution of multimers.

28. Plasmid, **characterized in that** it comprises:
a) an origin of replication and optionally a marker gene in the non-therapeutic part,
b) two sequences permitting a site-specific recombination, positioned in the direct orientation, and,
c) placed between said sequences b), a therapeutic part or expression cassette consisting of one or more genes of therapeutic, vaccinal, agricultural or veterinary interest, of an mrs sequence originating from the par locus of RK2, permitting the resolution of multimers, and a sequence capable of forming a triple helix by hybridization with a specific oligonucleotide.

29. Plasmid, **characterized in that** it comprises:
a) an origin of replication and optionally a marker gene in the non-therapeutic part,
b) two sequences permitting an integrase-dependent site-specific recombination, positioned in the direct orientation, and two sequences permitting a resolvase-dependent site-specific recombination, positioned next to the first two sequences and also in the direct orientation, and,
c) placed between said sequences b), a therapeutic part or expression cassette consisting of one or more genes of therapeutic, vaccinal, agricultural or veterinary interest and optionally of a sequence capable of forming a triple helix by hybridization with a specific oligonucleotide.

30. Plasmid according to claims 26, 28 and 29, **characterized in that** the sequence capable of forming a triple helix is defined as in claims 3 and 4.

31. Recombinant cell containing a plasmid or a recombinant DNA according to claim 13, 26, 28 or 29.

32. Recombinant cell comprising, inserted into its genome, one or more copies of a recombinant DNA according to claim 9.

33. Recombinant cell according to claim 31 or 32, **characterized in that** it is a bacterium.

34. Recombinant cell according to claim 31 or 32, **characterized in that** it is a eukaryotic cell.

35. Recombinant cell according to claim 33, **characterized in that** it is the bacterium E.coli D1 21 0HP.

36. Pharmaceutical composition comprising at least one DNA molecule according to one of claims 1 to 8.

37. Method for the preparation of a DNA molecule according to one of claims 1 to 8, **characterized in that** a culture of host cells containing a recombinant DNA as defined in claim 9 is brought into contact with the recombinase enabling site-specific recombination to be induced *in vivo*.

38. Method according to claim 37, **characterized in that** the culture of host cells is a culture of cells according to claim 31.

39. Method according to claim 37, **characterized in that** the culture of host cells is a culture of cells according to claim 32.

40. Method according to one of claims 37 to 39, **characterized in that** the culture is brought into contact with the recombinase by transfection or infection of the cell culture with a plasmid or a phage containing the gene for said recombinase.

41. Method according to one of claims 37 to 39, **characterized in that** the culture is brought into contact with the recombinase by induction of the expression of a gene coding for said recombinase, present in the host cell.

42. Method according to claim 41, **characterized in that** the host cell contains, integrated in its genome, the recombinase gene having a temperature-regulated expression, and the culture is brought into contact with the recombinase by culturing at the induction temperature.

43. Method according to claim 42, **characterized in that** the host cell used contains a lysogenic phage integrated in its genome, containing the gene for said recombinase.

44. Method for the preparation of a DNA molecule according to one of claims 1 to 8, **characterized in that** a preparation of recombinant DNA according to claim 11 is brought into contact with the recombinase enabling site-specific recombination to be induced in vitro.

45. Method according to claim 37 or 44, **characterized in that** it comprises an additional step of purification of the molecule according to one of claims 1 to 8.

46. Method according to claim 45, **characterized in that** the purification comprises a step of bringing a solution containing'the molecule accoding to one of claims 1 to 8 into contact with a specific ligand, optionally grafted onto a support.

47. Method according to claim 46, **characterized in that** the solution containing the molecule according to one of claims 1 to 8 is brought into contact with an oligonucleotide, optionally grafted onto a support, capable of forming, by hybridization, a triple helix with a specific sequence present in the said molecule according to one of claims 1 to 8.

## Patentansprüche

1. Doppelsträngiges DNA-Molekül, **dadurch gekennzeichnet, dass**:
- es in zirkulärer und supergeknäuelter Form vorliegt,
- es eine Expressionskassette umfasst, welche aus einem Gen von therapeutischem, Impf-, landwirtschaftlichem oder veterinärmedizinischem Interesse unter der Kontrolle eines Transkriptionspromotors und eines Transkriptionsterminators, welche in Säugetierzellen aktiv sind, besteht,
- es keinen Replikationsstartpunkt aufweist,
- es kein Markergen aufweist und
- es eine Region umfasst, welche aus der ortsspezifischen Rekombination zwischen zwei Sequenzen resultiert, wobei die Region außerhalb der Expressionskassette gelegen ist.

2. Molekül nach Anspruch 1, **dadurch gekennzeichnet, dass** es außerdem eine Sequenz enthält, welche in der Lage ist, durch Hybridisierung mit einem spezifischen Oligonukleotid eine Dreifachhelix zu bilden.

3. Molekül nach Anspruch 2, **dadurch gekennzeichnet, dass** die Sequenz, welche in der Lage ist, eine Dreifachhelix zu bilden, 5 bis 30 Basenpaare umfasst.

4. Molekül nach Anspruch 2 oder 3, **dadurch gekennzeichnet, dass** die Sequenz, welche in der Lage ist, eine Dreifachhelix zu bilden, eine Homopurin-Homopyrimidin-Sequenz ist.

5. Molekül nach Anspruch 1, **dadurch gekennzeichnet, dass** es eine Sequenz umfasst, welche aus der ortsspezifischen Rekombination zwischen zwei Anheftungssequenzen att oder zwischen zwei Erkennungssequenzen der Resolvase eines Transposons oder parA von RK2 resultiert.

6. Molekül nach einem der vorangegangenen Ansprüche, **dadurch gekennzeichnet, dass** es außerdem eine aus dem Genort par von RK2 stammende Sequenz mrs umfasst, welche erlaubt, Multimere aufzulösen.

7. Molekül nach einem der vorangegangenen Ansprüche, **dadurch gekennzeichnet, dass** das Gen von Interesse eine Nukleinsäure ist, welche ein therapeutisches, Impf-, landwirtschaftliches oder veterinärmedizinisches Produkt kodiert.

8. Molekül nach einem der vorangegangenen Ansprüche, **dadurch gekennzeichnet, dass** durch Herausschneiden ausgehend von einem Plasmid oder einem Chromosom, durch ortsspezifische Rekombination es erhalten wird.

9. Rekombinante DNA, umfassend einen nicht-therapeutischen Abschnitt und einen therapeutischen Abschnitt oder eine Expressionskassette, welche(r) aus einem Gen von therapeutischem, Impf-, landwirtschaftlichem oder veterinärmedizinischem Interesse unter der Kontrolle eines Transkriptionspromotors und eines Transkriptionsterminators, welche in Säugetierzellen aktiv sind, besteht und von zwei Sequenzen eingerahmt ist, welche eine ortsspezifische Rekombination erlauben und in direkter Orientierung angeordnet sind, wobei die Rekombination durch Herausschneiden der nicht-therapeutischen Abschnitte erlaubt, das Molekül nach einem der Ansprüche 1 bis 8 zu erzeugen.

10. Rekombinante DNA nach Anspruch 9, **dadurch gekennzeichnet, dass** es sich um ein replikatives Plasmid handelt, welches umfasst:
a) einen Replikationsstartpunkt und gegebenenfalls ein Markergen in dem nicht-therapeutischen Abschnitt,
b) zwei Sequenzen, welche eine ortsspezifische Rekombination erlauben und in direkter Orientierung angeordnet sind, und
c) platziert zwischen den Sequenzen b) einen therapeutischen Abschnitt oder eine Expressionskassette, welche(r) aus einem Gen von therapeutischem, Impf-, landwirtschaftlichem oder veterinärmedizinischem Interesse unter der Kontrolle eines Transkriptionspromotors und eines Transkriptionsterminators, welche in Säugetierzellen aktiv sind, besteht.

11. Rekombinante DNA nach den Ansprüchen 9 und 10, **dadurch gekennzeichnet, dass** die Sequenzen, welche die ortsspezifische Rekombination erlauben, Sequenzen sind, welche in der Lage sind, in Gegenwart einer Rekombinase spezifisch zu rekombinieren.

12. Rekombinante DNA nach Anspruch 11, **dadurch gekennzeichnet, dass** die Rekombinase ausgewählt wird unter den Rekombinasen der Familie der Integrase des Phagen lambda und der Familie der Resolvase des Transposons Tn3.

13. Rekombinante DNA nach einem der Ansprüche 9 bis 12, **dadurch gekennzeichnet, dass** die Sequenzen, welche die ortsspezifische Rekombination erlauben, von einem Bakteriophagen abgeleitet sind.

14. Rekombinante DNA nach Anspruch 13, **dadurch gekennzeichnet, dass** die Sequenzen, welche die ortsspezifische Rekombination erlauben, aus den Anheftungssequenzen att eines Bakteriophagen bestehen.

15. Rekombinante DNA nach Anspruch 14, **dadurch gekennzeichnet, dass** die Sequenzen, welche die ortsspezifische Rekombination erlauben, aus den Anheftungssequenzen des Bakteriophagen lambda, P22, Φ80, P1, HP1 oder ferner des Plasmids pSAM2 oder 2µ bestehen.

16. Rekombinante DNA nach Anspruch 15, **dadurch gekennzeichnet, dass** die Sequenzen, welche die ortsspezifische Rekombination erlauben, die Gesamtheit oder einen Teil der Sequenzen SEQ ID Nr. 1, 2, 6, 7, 8, 9, 10, 11, 12, 13 oder 14 umfassen.

17. Plasmid, **dadurch gekennzeichnet, dass** es umfasst:
(a) einen bakteriellen Replikationsstartpunkt und gegebenenfalls ein Markergen in dem nicht-therapeutischen Abschnitt;
(b) die Sequenzen attP und attB eines unter den Phagen lambda, P22, □80, P1, HP1 ausgewählten Bakteriophagen oder des Plasmids pSAM2 oder 2µ, welche in direkter Orientierung angeordnet sind; und
(c) platziert zwischen den Sequenzen einen therapeutischen Abschnitt oder eine Expressionskassette, welche(r) aus einem Gen von therapeutischem, lmpf-, landwirtschaftlichem oder veterinärmedizinischem Interesse unter der Kontrolle eines Transkriptionspromotors und eines Transkriptionsterminators, welche in Säugetierzellen aktiv sind, besteht.

18. Plasmid nach Anspruch 17, **dadurch gekennzeichnet, dass** die Sequenzen, welche die ortsspezifische Rekombination erlauben, aus den Anheftungssequenzen des Bakteriophagen lambda bestehen.

19. Rekombinante DNA nach Anspruch 13, **dadurch gekennzeichnet, dass** die Sequenzen, welche die ortsspezifische Rekombination erlauben, von dem Bakteriophagen P1 abgeleitet sind.

20. Plasmid, **dadurch gekennzeichnet, dass** es umfasst:
a) einen bakteriellen Replikationsstartpunkt und gegebenenfalls ein Markergen in dem nicht-therapeutischen Abschnitt,
b) die invertierten Sequenzenwiederholungen des Bakteriophagen P1 (Region loxP), welche in direkter Orientierung angeordnet sind; und
c) platziert zwischen den Sequenzen (b) einen therapeutischen Abschnitt oder eine Expressionskassette, welche(r) aus einem Gen von therapeutischem, Impf-, landwirtschaftlichem oder veterinärmedizinischem Interesse unter der Kontrolle eines Transkriptionspromotors und eines Transkriptionsterminators, welche in Säugetierzellen aktiv sind, besteht.

21. Rekombinante DNA nach einem der Ansprüche 9 bis 12, **dadurch gekennzeichnet, dass** die Sequenzen, welche die ortsspezifische Rekombination erlauben, von einem Transposon abgeleitet sind.

22. Rekombinante DNA nach Anspruch 21, **dadurch gekennzeichnet, dass** die Sequenzen, welche die ortsspezifische Rekombination erlauben, aus Erkennungssequenzen der Resolvase des Transposons Tn3, Tn21 oder Tn522 bestehen.

23. Rekombinante DNA nach Anspruch 22, **dadurch gekennzeichnet, dass** die Sequenzen, welche die ortsspezifische Rekombination erlauben, die Gesamtheit oder einen Teil der Sequenz SEQ ID Nr. 15 umfassen.

24. Rekombinante DNA nach einem der Ansprüche 9 bis 12, **dadurch gekennzeichnet, dass** die Sequenzen, welche die ortsspezifische Rekombination erlauben, von der Region par des Plasmids RP4 abgeleitet sind.

25. Rekombinante DNA nach einem der Ansprüche 9 bis 24, **dadurch gekennzeichnet, dass** sie außerdem eine Sequenz umfasst, welche in der Lage ist, durch Hybridisierung mit einem spezifischen Oligonukleotid eine Dreifachhelix zu bilden.

26. Plasmid, **dadurch gekennzeichnet, dass** es umfasst:
a) einen Replikationsstartpunkt und gegebenenfalls ein Markergen in dem nicht-therapeutischen Abschnitt,
b) zwei Sequenzen, welche eine ortsspezifische Rekombination erlauben und in direkter Orientierung angeordnet sind, und
c) platziert zwischen den Sequenzen b) einen therapeutischen Abschnitt oder eine Expressionskassette, welche(r) aus einem oder mehreren Genen von therapeutischem, Impf-, landwirtschaftlichem oder veterinärmedizinischem Interesse und einer Sequenz, welche in der Lage ist, durch Hybridisierung mit einem spezifischen Oligonukleotid eine Dreifachhelix zu bilden, besteht.

27. Plasmid, **dadurch gekennzeichnet, dass** es umfasst:
a) einen Replikationsstartpunkt und gegebenenfalls ein Markergen in dem nicht-therapeutischen Abschnitt,
b) zwei Sequenzen, welche eine ortsspezifische Rekombination erlauben und in direkter Orientierung angeordnet sind, und
c) platziert zwischen den Sequenzen b) einen therapeutischen Abschnitt oder eine Expressionskassette, welche(r) aus einem oder mehreren Genen von therapeutischem, Impf-, landwirtschaftlichem oder veterinärmedizinischem Interesse und einer aus dem Genort par von RK2 stammenden Sequenz mrs, welche erlaubt, Multimere aufzulösen, besteht.

28. Plasmid, **dadurch gekennzeichnet, dass** es umfasst:
a) einen Replikationsstartpunkt und gegebenenfalls ein Markergen in dem nicht-therapeutischen Abschnitt,
b) zwei Sequenzen, welche eine ortsspezifische Rekombination erlauben und in direkter Orientierung angeordnet sind, und
c) platziert zwischen den Sequenzen b) einen therapeutischen Abschnitt oder eine Expressionskassette, welche(r) aus einem oder mehreren Genen von therapeutischem, Impf-, landwirtschaftlichem oder veterinärmedizinischem Interesse, einer aus dem Genort par von RK2 stammenden Sequenz mrs, welche erlaubt, Multimere aufzulösen, und einer Sequenz, welche in der Lage ist, durch Hybridisierung mit einem spezifischen Oligonukleotid eine Dreifachhelix zu bilden, besteht.

29. Plasmid, **dadurch gekennzeichnet, dass** es umfasst:
a) einen Replikationsstartpunkt und gegebenenfalls ein Markergen in dem nicht-therapeutischen Abschnitt,
b) zwei Sequenzen, weiche eine ortsspezifische, Integrase-abhängige Rekombination erlauben und in direkter Orientierung angeordnet sind, und zwei Sequenzen, welche eine ortsspezifische, Resolvase-abhängige Rekombination erlauben und angrenzend an die beiden ersten und gleichfalls in direkter Orientierung angeordnet sind, und
c) platziert zwischen den Sequenzen b) einen therapeutischen Abschnitt oder eine Expressionskassette, welche(r) aus einem oder mehreren Genen von therapeutischem, Impf-, landwirtschaftlichem oder veterinärmedizinischem Interesse und gegebenenfalls einer Sequenz, welche in der Lage ist, durch Hybridisierung mit einem spezifischen Oligonukleotid eine Dreifachhelix Zu bilden, besteht.

30. Plasmid nach den Ansprüchen 26, 28 und 29, **dadurch gekennzeichnet, dass** die Sequenz, welche in der Lage ist, eine Dreifachhelix zu bilden, wie in den Ansprüchen 3 und 4 definiert ist.

31. Rekombinante Zelle, welche ein Plasmid oder eine rekombinante DNA nach Anspruch 13, 26, 28 oder 29 enthält.

32. Rekombinante Zelle, welche inseriert in ihr Genom eine oder mehrere Kopien einer rekombinanten DNA nach Anspruch 9 umfasst.

33. Rekombinante Zelle nach Anspruch 31 oder 32, **dadurch gekennzeichnet, dass** es sich um ein Bakterium handelt.

34. Rekombinante Zelle nach Anspruch 31 oder 32, **dadurch gekennzeichnet, dass** es sich um eine eukaryotische Zelle handelt.

35. Rekombinante Zelle nach Anspruch 33, **dadurch gekennzeichnet, dass** es sich um das Bakterium *E. coli* D1 21 0HP handelt.

36. Pharmazeutische Zusammensetzung, welche ein DNA-Molekül zumindest gemäß einem der Ansprüche 1 bis 8 umfasst.

37. Verfahren zur Herstellung eines DNA-Moleküls nach einem der Ansprüche 1 bis 8, **dadurch gekennzeichnet, dass** eine Kultur von Wirtszellen, welche eine rekombinante DNA, wie in Anspruch 9 definiert, enthalten, mit der Rekombinase, welche erlaubt, *in vivo* die ortsspezifische Rekombination zu induzieren, in Kontakt gebracht wird.

38. Verfahren nach Anspruch 37, **dadurch gekennzeichnet, dass** die Kultur von Wirtszellen eine Kultur von Zellen nach Anspruch 31 ist.

39. Verfahren nach Anspruch 37, **dadurch gekennzeichnet, dass** die Kultur von Wirtszellen eine Kultur von Zellen nach Anspruch 32 ist.

40. Verfahren nach einem der Ansprüche 37 bis 39, **dadurch gekennzeichnet, dass** das Inkontaktbringen mit der Rekombinase ausgeführt wird durch Transfektion oder Infektion der Kultur von Zellen mit einem Plasmid oder einem Phagen, welches bzw. welcher das Gen der Rekombinase enthält.

41. Verfahren nach einem der Ansprüche 37 bis 39, **dadurch gekennzeichnet, dass** das Inkontaktbringen mit der Rekombinase ausgeführt wird durch Induktion der Expression eines in der Wirtszelle vorhandenen Gens, welches die Rekombinase kodiert.

42. Verfahren nach Anspruch 41, **dadurch gekennzeichnet, dass** die Wirtszelle integriert in ihr Genom das Gen der Rekombinase enthält, welches eine wärmeregulierte Expression aufweist, und das Inkontaktbringen mit der Rekombinase durch Kultivierung bei der Induktionstemperatur erfolgt.

43. Verfahren nach Anspruch 42, **dadurch gekennzeichnet, dass** die eingesetzte Wirtszelle einen in ihr Genom integrierten lysogenen Phagen enthält, welcher das Gen der Rekombinase enthält.

44. Verfahren zur Herstellung eines DNA-Moleküls nach einem der Ansprüche 1 bis 8, **dadurch gekennzeichnet, dass** eine Präparation von rekombinanter DNA nach Anspruch 11 mit der Rekombinase, welche erlaubt, die ortsspezifische Rekombination in vitro zu induzieren, in Kontakt gebracht wird.

45. Verfahren nach Anspruch 37 oder 44, **dadurch gekennzeichnet, dass** es einen ergänzenden Schritt einer Reinigung des Moleküls nach einem der Ansprüche 1 bis 8 umfasst.

46. Verfahren nach Anspruch 45, **dadurch gekennzeichnet, dass** die Reinigung einen Schritt des Inkontaktbringens einer Lösung, welche das Molekül nach einem der Ansprüche 1 bis 8 enthält, mit einem spezifischen Liganden umfasst, welcher gegebenenfalls auf einen Träger aufgepfropft ist.

47. Verfahren nach Anspruch 46, **dadurch gekennzeichnet, dass** die Lösung, welche das Molekül nach einem der Ansprüche 1 bis 8 enthält, mit einem Oligonukleotid in Kontakt gebracht wird, welches gegebenenfalls auf einem Träger aufgepfropft ist und in der Lage ist, durch Hybridisierung eine Dreifachhelix mit einer in dem Molekül nach einem der Ansprüche 1 bis 8 vorhandenen spezifischen Sequenz zu bilden.
